(19) Europäisches Patentamt
European Patent Office
Office européen des brevets

(11) **EP 3 862 109 A2**

(12) **EUROPEAN PATENT APPLICATION**
published in accordance with Art. 153(4) EPC

(43) Date of publication:
**11.08.2021 Bulletin 2021/32**

(21) Application number: **19794172.7**

(22) Date of filing: **25.09.2019**

(51) Int Cl.:
**B22F 1/00** *(2006.01)*     **A61P 35/00** *(2006.01)*
**A61K 33/38** *(2006.01)*

(86) International application number:
**PCT/ES2019/070637**

(87) International publication number:
**WO 2020/065115 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2018 EP 18196959**
**26.09.2018 EP 18196963**

(71) Applicants:
• **Nanogap Sub NM Powder, S.A.**
**15895 Ames La Coruña (ES)**

• **Universidade de Santiago de Compostela**
**15782 Santiago de Compostela, La Coruña (ES)**

(72) Inventors:
• **BUCETA FERNÁNDEZ, David**
**15895 Ames - A Coruña (ES)**
• **DOMÍNGUEZ PUENTE, Fernando**
**15895 Ames - A Coruña (ES)**
• **LÓPEZ QUINTELA, Manuel Arturo**
**15895 Ames - A Coruña (ES)**

(74) Representative: **Teuten, Andrew John**
**Sagittarius IP**
**Marlow International**
**Parkway**
**Marlow SL7 1YL (GB)**

(54) **THERAPEUTIC USES OF ATOMIC QUANTUM CLUSTERS**

(57)     There is provided an invention relating to compositions and therapeutic uses of atomic quantum clusters (AQCs), in particular compositions consisting essentially of AQCs comprising 5 zero-valent transition metal atoms for use in the treatment of a cell proliferative disorder.

EP 3 862 109 A2

**Description**

**FIELD OF THE INVENTION**

The invention relates to therapeutic uses of atomic quantum clusters, in particular atomic quantum clusters consisting of 5 zero-valent transition metal atoms.

**BACKGROUND OF THE INVENTION**

Redox homeostasis is essential for cell survival. Thiols play a central role in the maintenance of redox balance. The sulphur atom in the side-chain of the amino acid cysteine can exist in several different oxidation states. Under physiological conditions, cysteine's sulphur atom reversibly transits between thiol and disulphide states (reduced and oxidized, respectively) but transition into higher oxidation states (except for sulphonic acid) is irreversible, meaning that the protein can only be replaced by the synthesis of a new one. Cells in their different compartments, with the only exception of the endoplasmic reticulum, are continuously reducing proteins that are spontaneously oxidized by the presence of oxygen. Inside the cell, protein functions are dependent on their sulphur oxidation state. There are two overlapping systems, the glutathione and thioredoxin systems, which are very well-preserved throughout evolution, and work to keep protein cysteines in their functional, reduced state.

Reactive oxygen species (ROS) are generated during normal metabolism of cells and the glutathione and thioredoxin systems protect cells from oxidative damage by maintaining the reduced state. If ROS levels are elevated and exceed the buffering capacity of the glutathione and thioredoxin systems, activation of signalling pathways and gene expression can occur which induces cell apoptosis. Active proliferating tumour cells show increased respiration and, as consequence, higher ROS levels. Moreover, human tumours show insufficient vascularization that contribute to glucose starvation and ROS increase due to an imbalance of redox homeostasis.

WO2012/059572 describes a combination of at least one AQC and at least one antineoplastic drug for the prevention and/or treatment of a cell proliferative disorder. The application describes AQCs consisting of between 2 and 25 zero-valent transition metal atoms having a cytotoxic and anti-proliferative effect on cancer cell lines and therefore may be used in combination with antineoplastic agents to treat cell proliferative disorders.

It is an object of the invention to provide improved therapeutic compositions of AQCs.

**SUMMARY OF THE INVENTION**

In a first aspect, the invention provides a composition comprising atomic quantum clusters (AQCs) consisting of 5 zero-valent transition metal atoms for use in the treatment of a cell proliferative disorder.

In another aspect, the invention provides the use of the composition as defined herein, for the preparation of a pharmaceutical composition for the treatment of a cell proliferative disorder.

In another aspect, the invention provides the use of a composition comprising atomic quantum clusters (AQCs) consisting of 5 zero-valent transition metal atoms as a radiation therapy sensitizing agent for proliferating cells.

In another aspect, the invention provides a method of preventing or treating a cell proliferative disorder comprising administering a therapeutically effective amount of the composition as defined herein, to a patient in need thereof.

In another aspect, the invention provides a method of preventing or treating a cell proliferative disorder comprising administering a therapeutically effective amount of a composition comprising atomic quantum clusters (AQCs) consisting of 5 zero-valent transition metal atoms, to a patient in need thereof, wherein said method does not comprise treating the patient with an additional antineoplastic drug.

In another aspect, the invention provides a method of preventing or treating a cell proliferative disorder comprising administering a therapeutically effective amount of a composition comprising atomic quantum clusters (AQCs) consisting of 5 zero-valent transition metal atoms, to a patient in need thereof, in combination with radiation therapy.

These and other aspects are described in more detail in the following description.

**BRIEF DESCRIPTION OF THE FIGURES**

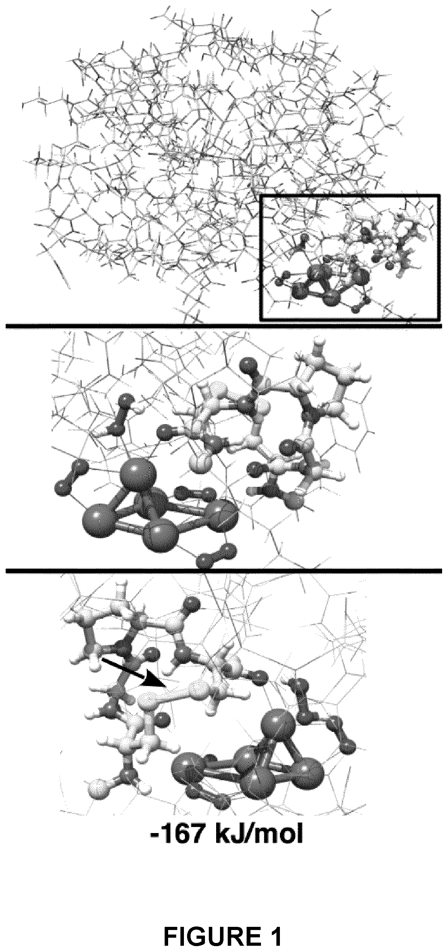

**Figure 1:** Interaction of Ag5-AQC with *E. Coli* thioredoxin. As seen, Ag5-AQC (large, grey, five-membered molecule) binds to the cysteine (molecules highlighted by a black arrow) residues that form the active site. The energy of the binding is favourable (-167 kJ/mol).

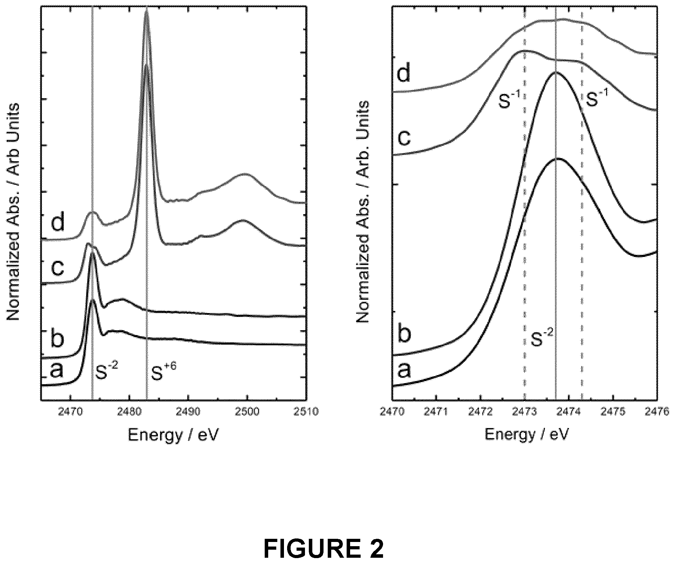

**Figure 2:** Normalised Sulphur K edge X-ray absorption near edge structure (S-K XANES) spectra of: cysteine (a) and glutathione (b) and the corresponding ones with addition of Ag5-AQCs (c & d, respectively). Right panel shows the augmented region of the left-hand graph. Vertical full and dashed lines indicate the energy position associated to the different S-oxidation states.

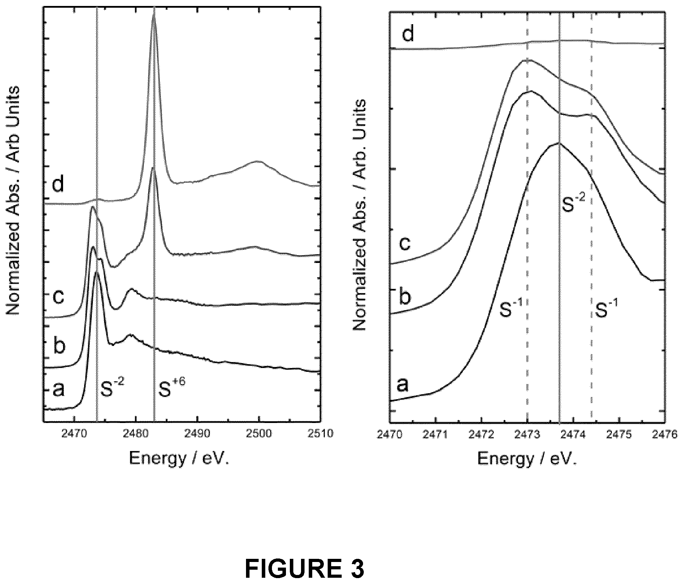

**Figure 3:** Normalised S-K XANES spectra of: (a) cysteine and cysteine treated with Ag5-AQC at difference concentrations: (b) 1:106 diluted respect to reference stock concentration (RSC), (c) 1:103 diluted respect to RSC and (d) RSC. Vertical lines indicate the corresponding energy for different S-oxidation states.

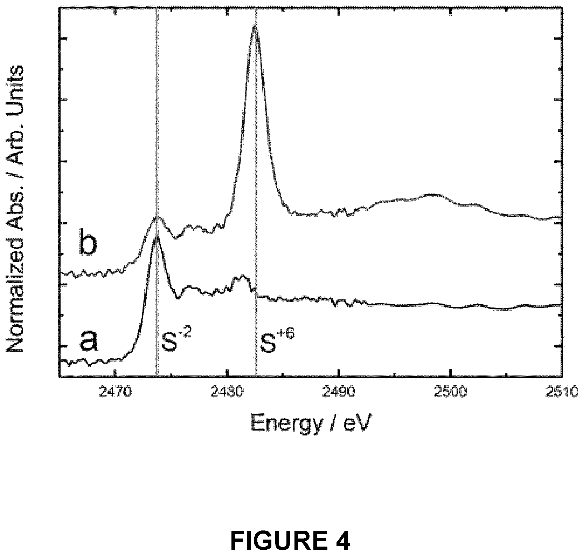

**Figure 4:** Normalised S-K XANES spectra of: thioredoxin in water solution with PBS (a) before and (b) after treatment with Ag5-AQC. Vertical lines indicate the corresponding energy for different S-oxidation states.

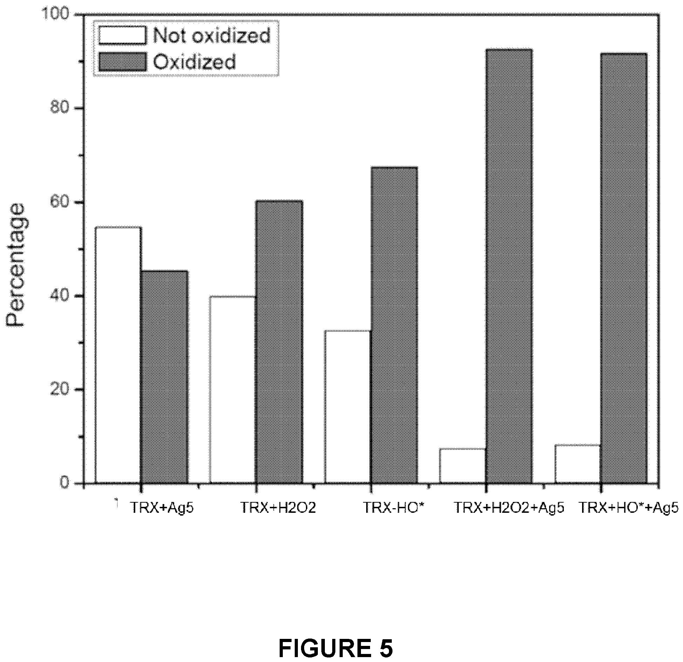

**Figure 5:** Percentage of thioredoxin (TRX) oxidized with Ag5-AQCs, hydrogen peroxide ($H_2O_2$) and hydroxyl radical (HO•) alone or in various combinations, as shown on the x axis.

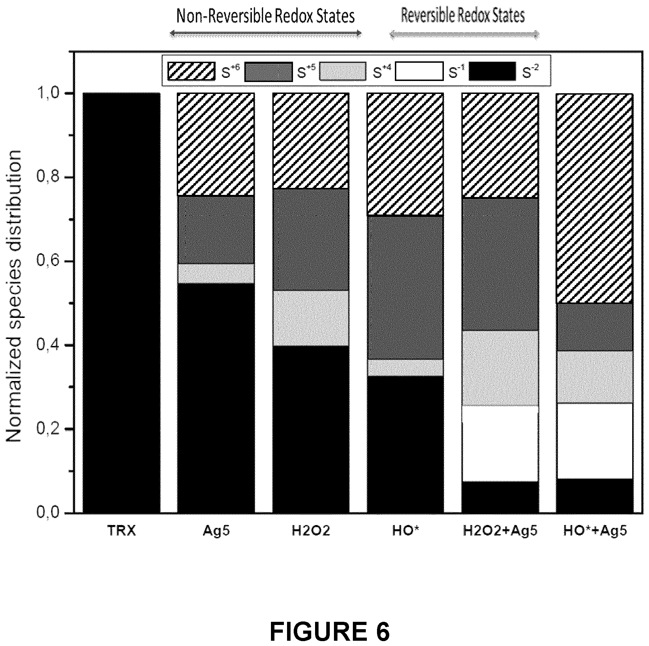

**Figure 6:** Sulphur oxidation number of thioredoxin cysteines after various treatments. Sulphur oxidation is affected by Ag5-AQCs, hydrogen peroxide ($H_2O_2$) and hydroxyl radical (HO•). The combination with Ag5-AQC greatly potentiates the effect of $H_2O_2$ and HO•.

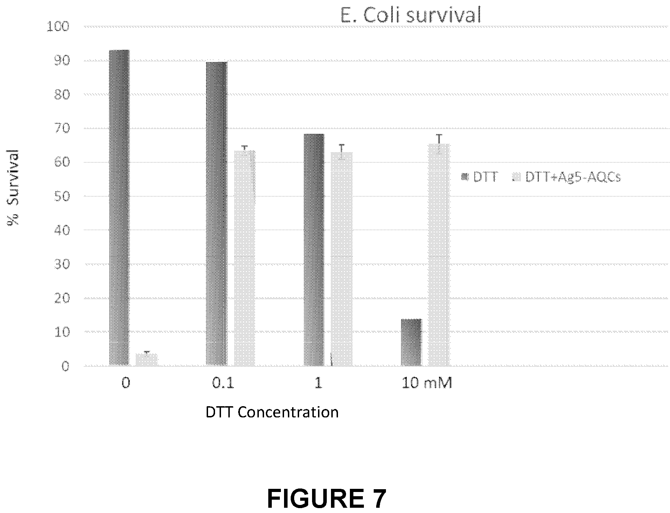

**Figure 7:** *E. Coli* survival was measured after addition of different concentrations of dithiothreitol (DTT) either alone (control) or in combination with Ag5-AQC. In absence of DTT (0 mM) a low concentration of Ag5-AQC kill the bacteria. When an increased concentration of DTT (0.1 mM) is co-administered with Ag5-AQC, bacteria viability is partially restored indicating that DTT rescues *E. Coli* from Ag5-AQC action. Correspondingly, DTT at 10mM is toxic for bacteria, however co-administration with Ag5-AQC reverts the DTT effect.

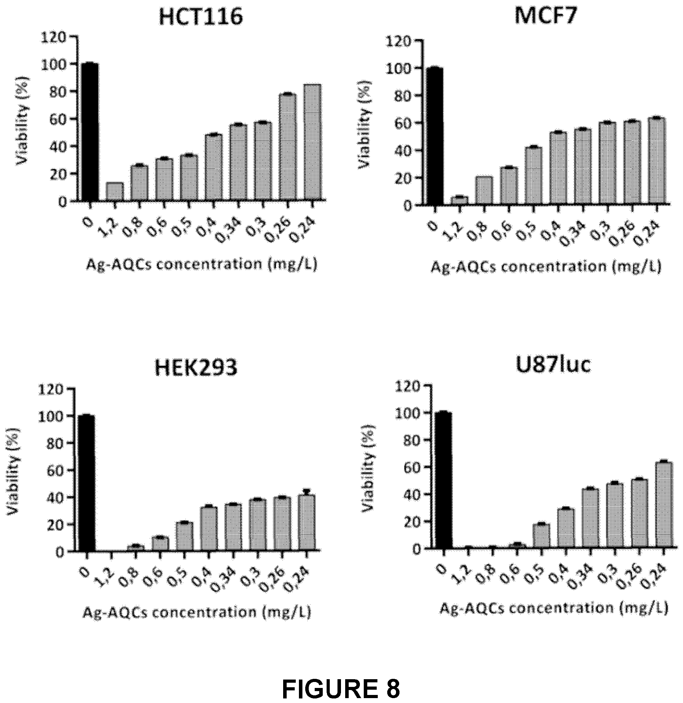

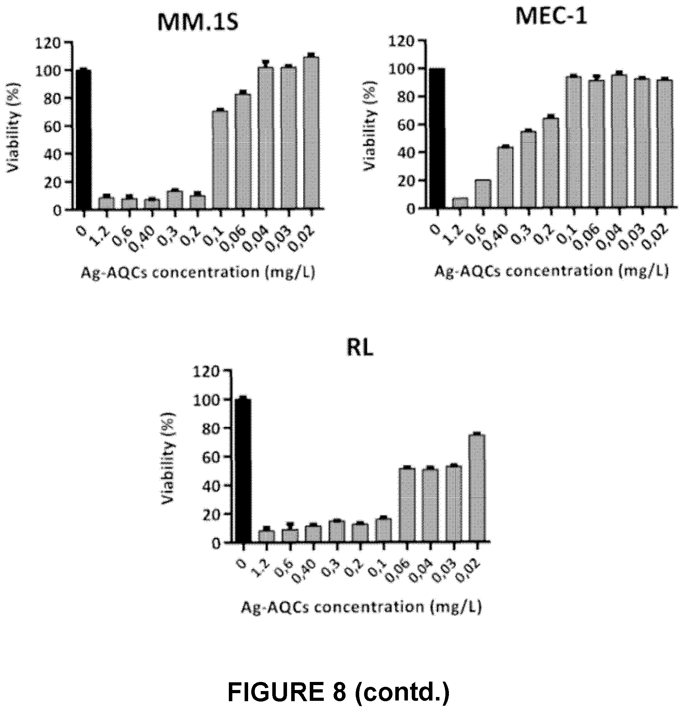

**Figure 8:** Dose-response (0.24-1.2 mg/L) graphs for various cell lines upon addition of Ag5-AQCs.

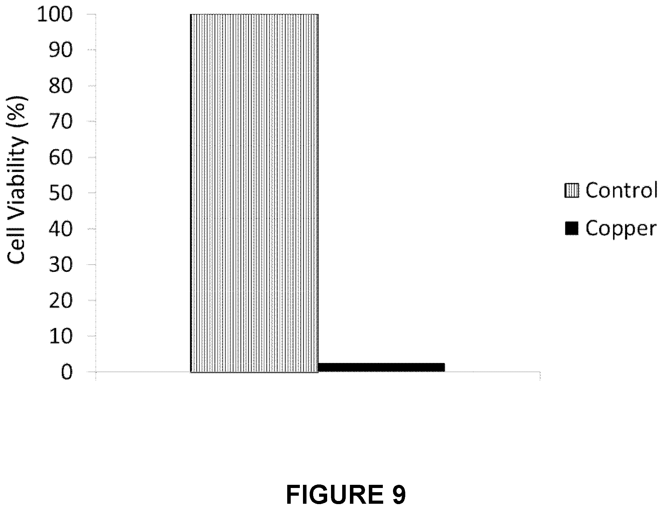

**Figure 9:** Results showing percentage cell viability of an A549 cell line upon addition of 5 atom clusters made of copper (Cus-AQCs) when compared to a control.

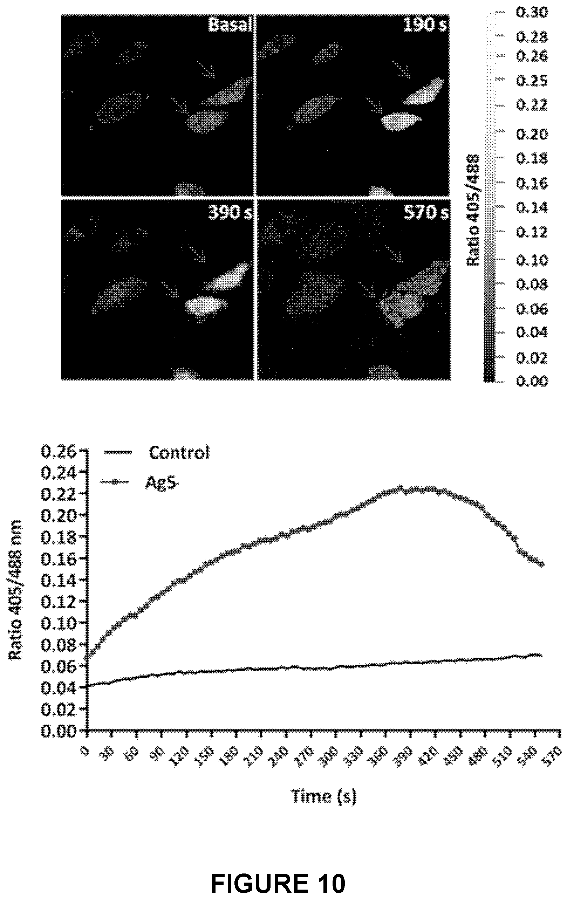

**Figure 10: Ag5-AQC oxidization of sulfhydryl groups in proteins.** A549 cells were transduced with Premo Cellular Redox Sensor. After 48 hours, time lapse imaging was performed using a Leica TCS SP5 confocal microscope. Samples were excited with 405 and 488 nm lasers, and the ratio of emissions in the green channel (500-530 nm) was calculated (ratio 405/488). Images were taken every 10 seconds after the addition of Ag5-AQC (IC50) during 10 minutes. False-colour ratio pictures of the cells at indicated time points highlight the changes in redox state. In each experiment, the ratio was quantified for two individual cells (arrowheads) and plotted against time.

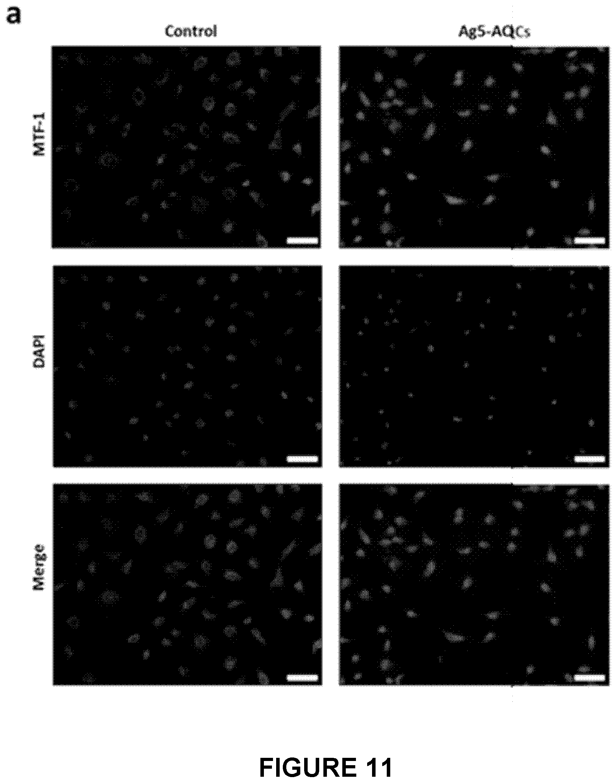

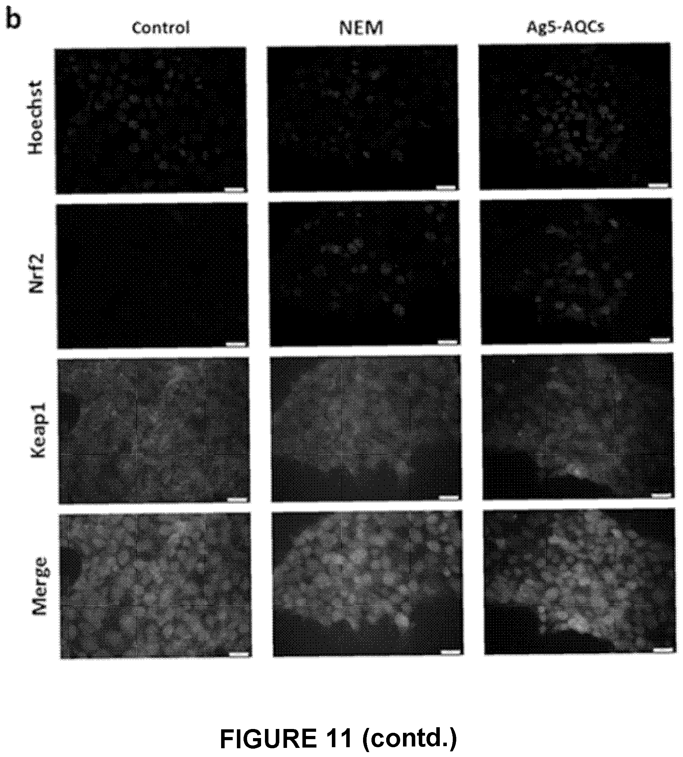

**Figure 11:** (a) MTF1 location in response to Ag5-AQCs was detected by indirect immunofluorescence. A549 cells were treated with Ag5-AQCs for 1 hour and 2 hours and later fixed and stained with the anti-MTF1 antibody and DAPI to counterstain the nucleus. Ag5-AQCs showed a clear translocation of MTF-1 into the nucleus (right column) compared to control cells (left column) (b) Ag5-AQCs induce Nrf2 translocation from cytoplasm to nucleus in HEK293 cells. Immunofluorescence staining was performed using an anti-Nrf2 antibody (red) and an anti-Keap1 antibody (green). Nuclei were counterstained with Hoechst (blue). Merged images show the nuclear location of Nrf2 after 30 minutes of treatment with Ag5-AQCs (IC50) or N-Ethylmaleimide (NEM) (100 $\mu$M, positive control).

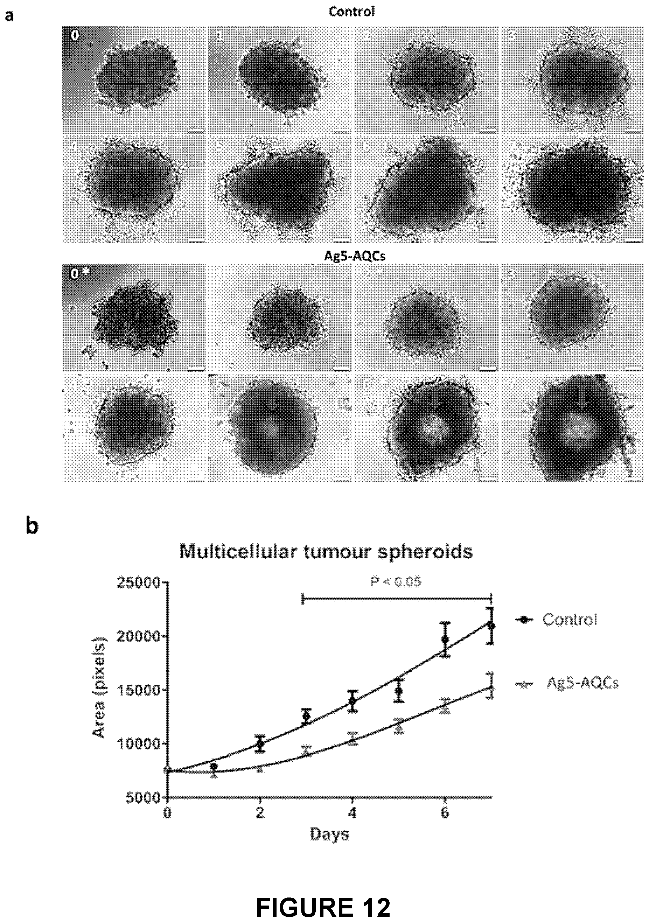

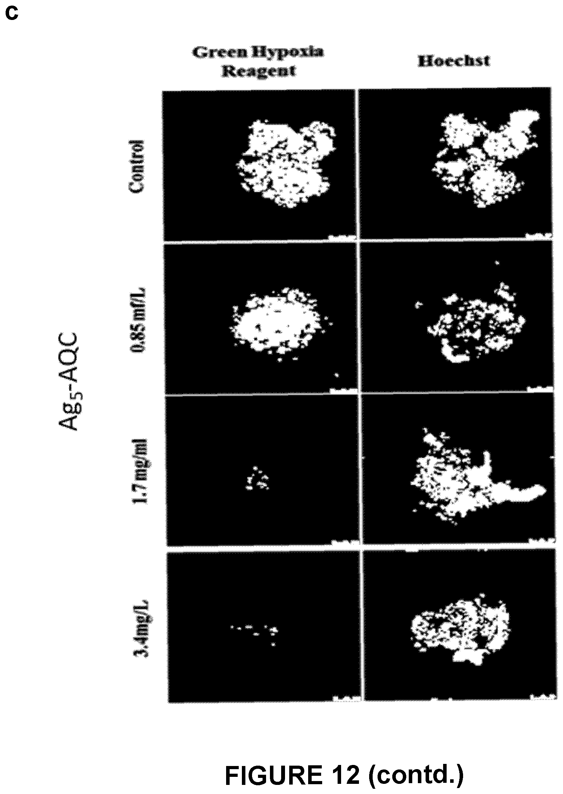

**Figure 12: Ag5-AQC treatment reduces A549 multicellular tumour spheroid (MCTS) size.** (a) Images of MCTS control and treated with Ag5-AQCs showing differences in MCTS size and cellular density in central regions. White asterisk indicates the day of treatment and arrows point to central regions with less cellular density as a result of Ag5-AQCs treatment (b) Growth kinetics of MCTS control or treated with Ag5-AQCs. Data represents mean $\pm$ SD. Error bars represent standard deviation; n=8. Mann Whitney test ((*) p <0.05). (c) Images using hypoxia agent and Hoechst staining to show the level of hypoxia in the tumouroids with or without (control) Ag5-AQC treatment.

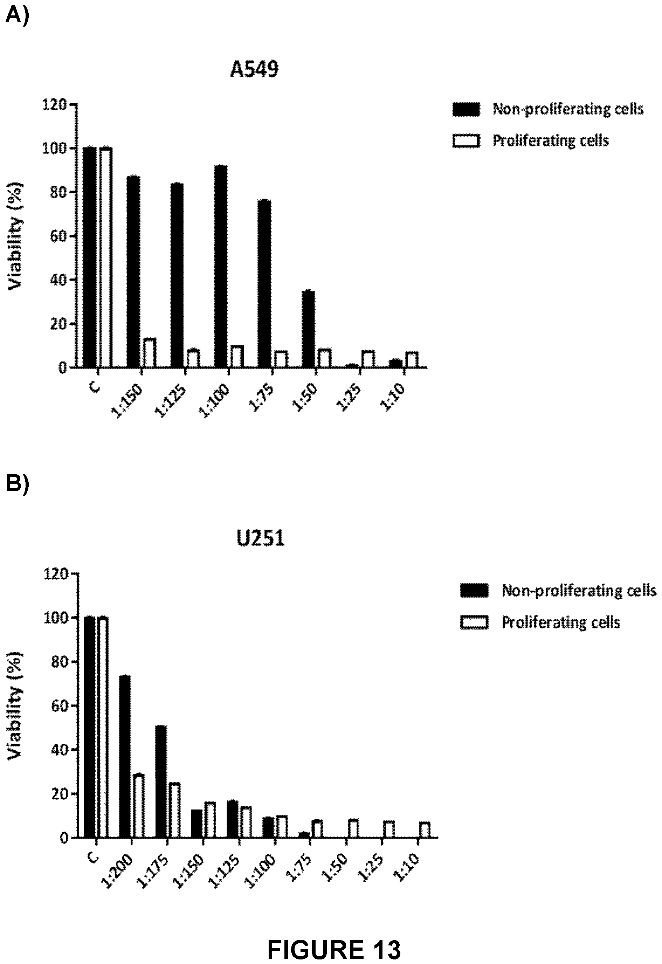

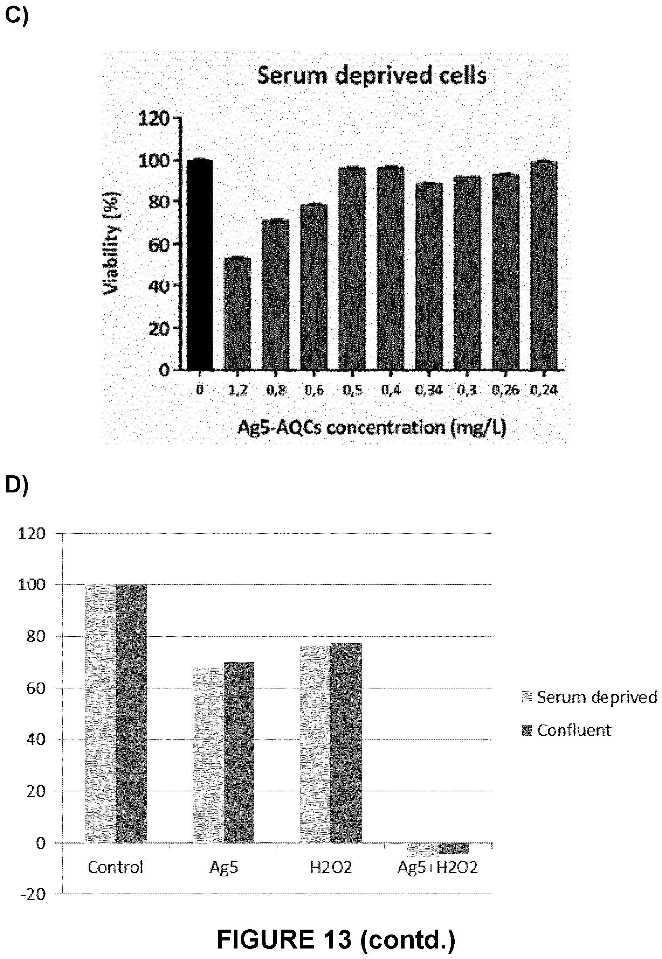

**Figure 13: Proliferating cells are more sensitive to the effect of Ag5-AQCs than non-proliferating cells.** (A) Proliferating and non-proliferating A549 cells, (B) proliferating and non-proliferating U251 cells, and (C) serum deprived A549 cells were exposed to different concentrations of Ag5-AQC for 1 hour and cell viability was determined by MTT assay. Data are shown as the mean $\pm$ SD of three independent experiments. D) Serum deprived or confluent A549 cells were treated with 1.2 mg/L of Ag5-AQC alone or in combination with $H_2O_2$. A synergistic effect is clearly seen when Ag5-AQC and $H_2O_2$ are co-administered.

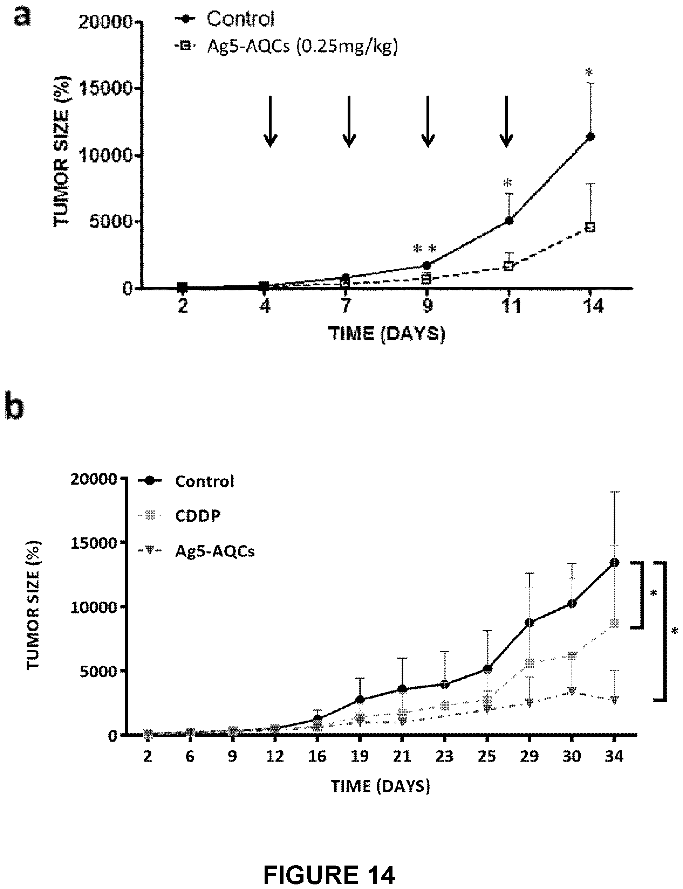

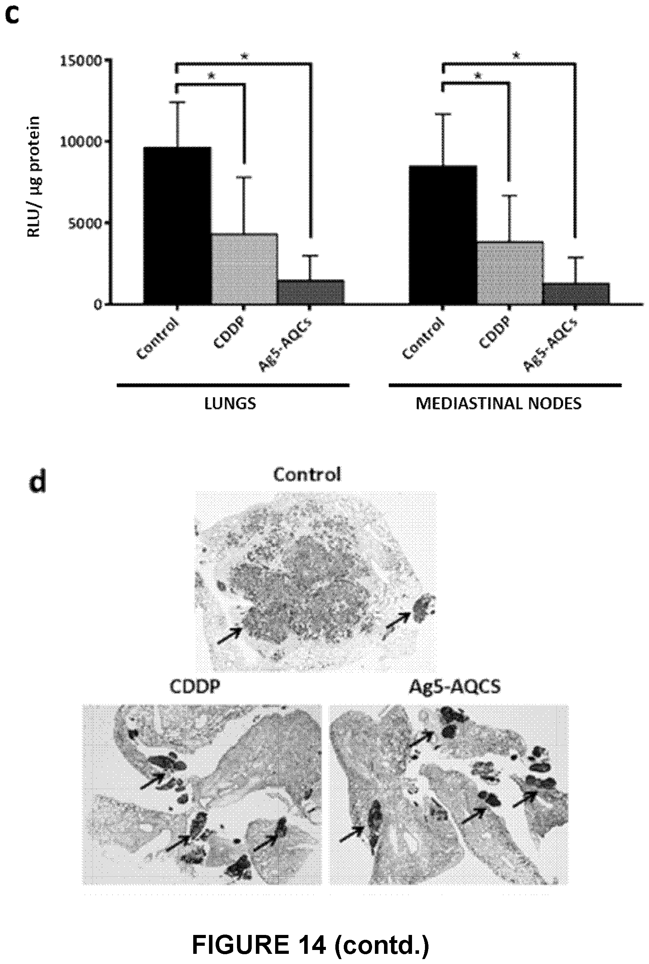

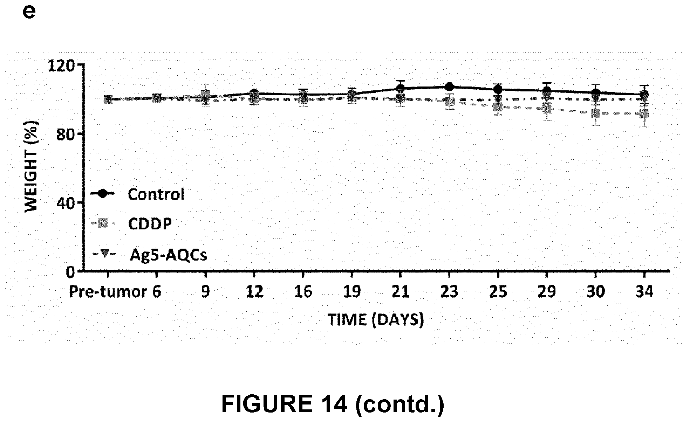

**Figure 14: Ag5-AQC *in vivo* effects,** (a) Ag5-AQCs cause a reduction in tumour growth in mice with orthotopic brain cancer. Experimental groups: Ag5-AQCs (0.25 mg/kg) and control (no treatment). (b-d) Ag5-AQCs treatment causes a reduction in tumour growth in mice with orthotopic lung cancer. (b) Tumour growth measured *in vivo* by luminescence (IVIS® Spectrum). Black arrows represent treatment administration times in the study. (c) Luciferase activity quantification measured *ex vivo* in lung and in mediastinal lymph nodes. (d) Immunohistochemical staining of lung tumour (arrows indicate the tumour nodules). Experimental groups: CDDP (4 mg/kg), Ag5-AQCs (0.25 mg/kg) and control (no treatment). (e) Body weight over the whole experiment. Experimental groups: CDDP (4 mg/kg), Ag5-AQCs (0.25 mg/kg) and control (no treatment). Data represents mean $\pm$ SD. Error bars represent standard deviation; n=5. Mann Whitney test ((*) p <0.05).

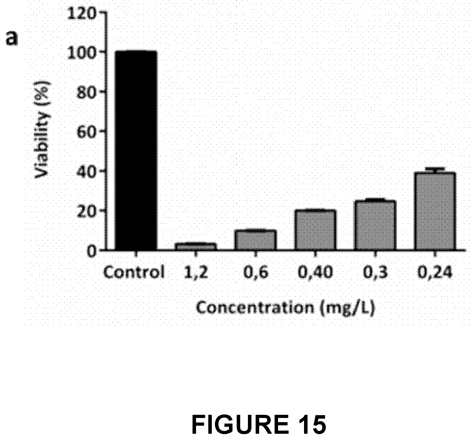

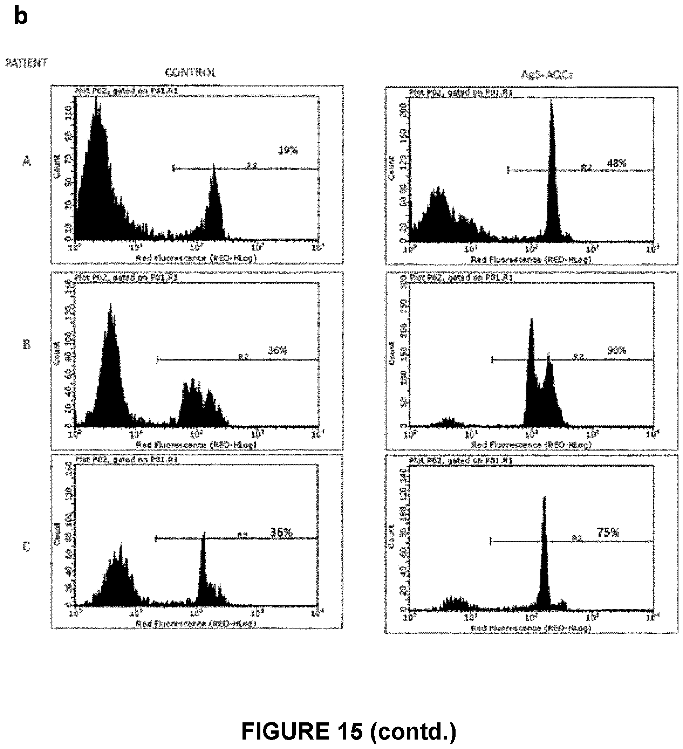

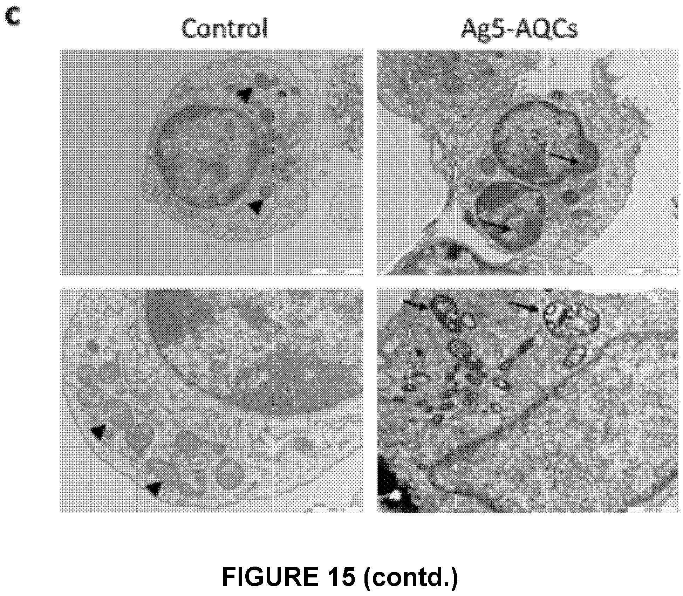

**Figure 15: Ag5-AQC treatment causes a reduction in cell viability in B-CLL cells derived from patients.** (a) Concentration-dependent reduction in B-CLL primary cells viability after Ag5-AQC treatment. Cells were exposed to different concentrations of Ag5-AQC for 30 minutes and cell viability was assessed after 24 hours by the MTT assay. (b) Ag5-AQC treatment increases the percentage of DHE positive cells 2.5 fold compared to control. B-CLL cells were treated with Ag5-AQC for 1 hour and 4 hours later DHE positive cells were quantified by flow cytometry. (c) TEM images of B-CLL cells treated with Ag5-AQC showed evident signs of apoptosis such as chromatin marginalization (black arrows) and disrupted mitochondria (black arrows).

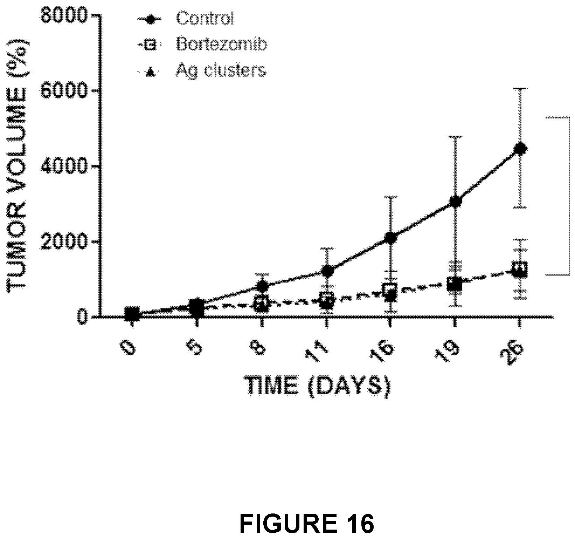

**Figure 16: Ag5-AQC treatment causes a reduction in tumour volume in multiple myeloma xenograft model.** Tumour volume was measured over the course of 26 days between three different treatment groups: Control (saline solution administered intravenously, n=4), Ag5-AQCs (0.0125 mg/kg administered intravenously, n=4), and Bortezomib (0.25 mg/kg administered intraperitoneally , n=4).

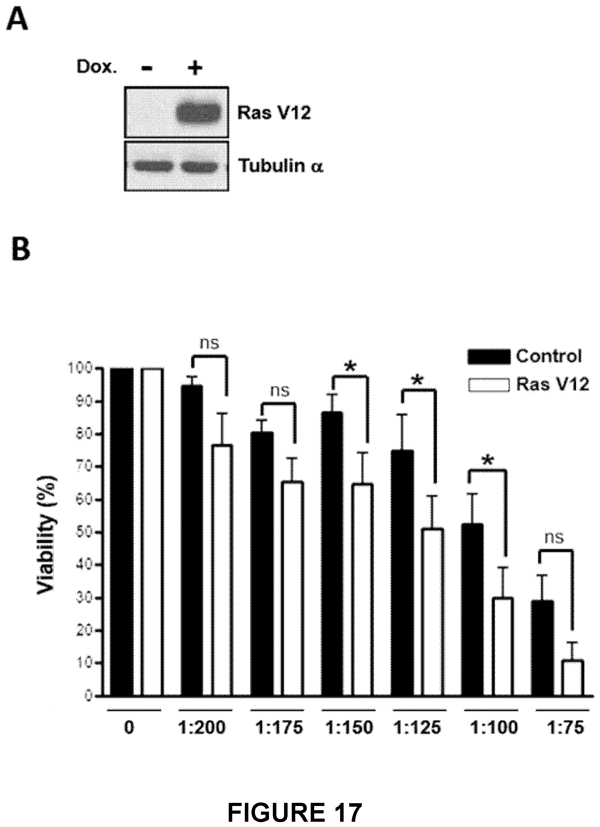

**Figure 17:** W3T3 cells carrying a doxycycline-inducible RasV12 allele were exposed doxycycline (white bars) or vehicle (control - black bars) for 24 hours and then treated with different concentrations of Ag5-AQCs. (a) RasV12 expression was assessed by western blot upon addition of doxycycline and (b) cell viability was determined by MTT assay 24 hours later. Data are shown as the mean $\pm$ SD of at least three independent experiments.

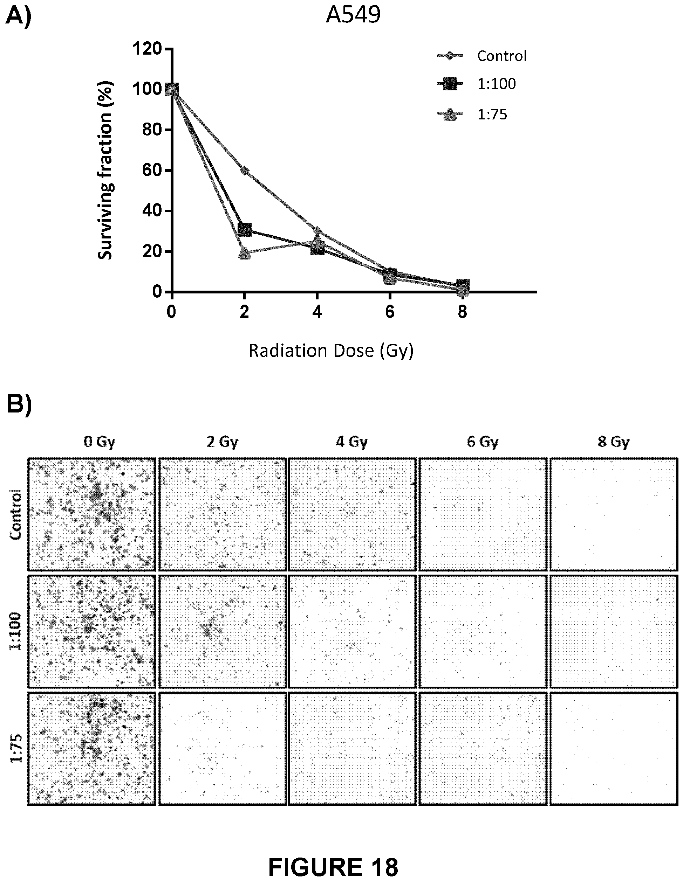

**Figure 18: Ag5-AQC treatment increases A549 cell sensitivity to radiation.** Results of the effect of radiation in A549 cells treated with Ag5-AQCs shown in A) a clonogenic assay and B) a DNA damage assay using anti-pH2AX staining.

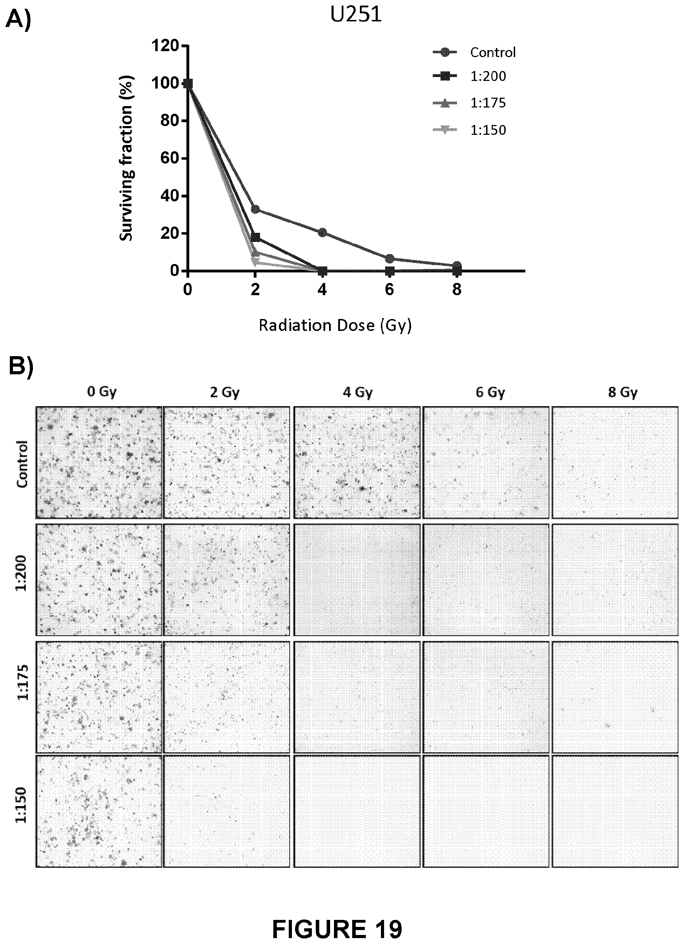

**Figure 19: Ag5-AQC treatment increases U251 cell sensitivity to radiation.** Results of the effect of radiation in U251 cells treated with Ag5-AQCs shown in A) a clonogenic assay and B) a DNA damage assay using anti-pH2AX staining.

## DETAILED DESCRIPTION OF THE INVENTION

### Definitions

All technical and scientific terms used throughout the specification have the same meaning as those commonly understood by a person skilled in the art.

Throughout the specification, use of the term "about" with respect to any quantity is contemplated to include that quantity.

Throughout the specification, unless the context requires otherwise, the word "comprise", and variations such as "comprises" and "comprising", will be understood to imply the inclusion of a stated integer, step, group of integers or group of steps but not to the exclusion of any other integer, step, group of integers or group of steps.

Throughout the specification, unless the context requires otherwise, the term "consisting essentially of", and variations such as "consists essentially of", will be understood to imply the inclusion of a stated integer, step, group of integers or group of steps, with the exclusion of any other integer, step, group of integers or group of steps which materially affects the essential characteristics of the stated integer, step, group of integers or group of steps.

Throughout the specification, unless the context requires otherwise, the term "consisting of", and variations such as "consists of", will be understood to imply the inclusion of a stated integer, step, group of integers or group of steps, with the exclusion of any other integer, step, group of integers or group of steps.

The term "atomic quantum clusters" or "AQCs" as used herein, refers to a group/cluster of 2 to 500 zero-valent transition metal atoms, such as between 2 to 200, 2 to 100, 2 to 50 or 2 to 25 transition metal atoms, and with a size less than 2 nm, such as less than 1 nm. The AQCs may comprise zero-valent transition metal atoms of identical (mononuclear clusters) or different (heteronuclear clusters) transition metals. It will be understood that this term does not include metal ions.

The term "transition metal" will be understood to refer to the elements of the periodic table known as transition metals, but it does not refer to the electrical behaviour of said elements. The confinement of electrons in the AQCs originates the quantum separation of the energy levels producing important changes in the properties of these materials, as reported in EP1914196. Thus, the metal atoms in the AQCs described herein can have a semiconductor-like or even insulating-like behaviour.

The term "substantially free of" may be used to refer to a composition which is mostly or completely free of an entity specifically mentioned thereafter, or at least does not contain the entity in an amount such that the entity affects the efficacy, storability, usability regarding necessary safety concerns, and/or stability of the composition.

The term "treat", "treating" or "treatment" may include prophylaxis and means to ameliorate, alleviate symptoms, eliminate the causation of the symptoms either on a temporary or permanent basis, or to prevent or slow the appearance of symptoms of the named disorder or condition. The compositions of the invention are useful in the treatment of humans and non-human animals.

The term "effective amount", "therapeutically effective amount" or "effective dose" refers to the amount sufficient to elicit the desired pharmacological or therapeutic effects, thus resulting in effective prevention or treatment of the disorder. Prevention of the disorder is manifested by delaying the onset of the symptoms of the disorder to a medically significant extent. Treatment of the disorder is manifested by a decrease in the symptoms associated with the disorder or an amelioration of the reoccurrence of the symptoms of the disorder.

**Compositions**

The present inventors herein provide evidence that atomic quantum clusters (AQCs) consisting of 5 zero-valent transition metal atoms affect both the glutathione and thioredoxin systems, thus affecting cell viability. A fundamental feature of the action of AQCs with 5 atoms on biological systems is their specificity for both substrates, proteins and electron acceptors. Theoretical and experimental evidence is provided of the interaction of Ag5-AQC with cysteine, glutathione and thioredoxin. And, importantly, the inventors also provide evidence that Ag5-AQCs are biologically dependent upon the presence of electron acceptors as shown by the fact that the activity of Ag5-AQC is greater with hydroxyl radical (HO•) > $H_2O_2$ > $O_2$ (*e.g.* **Figures 5 & 6).** Without being bound by theory, the evidence presented herein suggests that AQCs with 5 atoms increase the effect of ROS by acting as a catalytic bridge between ROS and sulphur atoms in proteins to increase the level of thiol oxidation. This mechanism of action is distinct to other chemotherapeutic drugs currently known in the art. The mechanism of action for AQCs of 5 atoms is demonstrated *in vitro,* in cell culture in 2D and 3D, animal models and primary cultures of tumour cells obtained from patients.

Therefore, according to a first aspect of the invention, there is provided a composition a composition comprising atomic quantum clusters (AQCs) consisting of 5 zero-valent transition metal atoms for use in the treatment of a cell proliferative disorder.

The potential therapeutic uses of compositions comprising AQCs consisting of 5 zero-valent transition metal atoms will be described herein. It has surprisingly been found that such compositions have a cytotoxic effect on eukaryotic cells on their own, without the need for additional antineoplastic agents to be present. Theoretical and experimental evidence provided herein shows that AQCs consisting of 5 atoms selectively interact with cysteine residues present in proteins and result in sulphur oxidation in the presence of Reactive Oxygen Species (ROS). This mechanism is unique to clusters of this size. Therefore, this application provides, for the first time, the motivation to use AQCs with 5 zero-valent transition metal atoms as a monotherapy in the treatment of cell proliferation diseases, such as cancer.

The composition may consist essentially of atomic quantum clusters (AQCs) for use in the treatment of a cell proliferative disorder, wherein said composition comprises AQCs consisting of 5 zero-valent transition metal atoms. In one embodiment, said AQCs are the sole active ingredient of the composition, *i.e.* no further active ingredients are present in the composition.

In one embodiment, the composition does not comprise an antineoplastic drug. In a further embodiment, the composition does not comprise an antineoplastic drug as described in WO2012/059572, such as alkylating agents *(e.g.* nitrogen mustard analogues, nitrosoureas, alkyl sulfonates, platinum containing compounds, ethylemines, and imidazotetrazines), cytotoxic antibiotics *(e.g.* anthracyclines, actinomycins), plant alkaloids and other natural products *(e.g.* campthotecin derivatives, epipodophyllotoxins, taxanes, and vinca alkaloids), antimetabolites *(e.g.* cytidine analogues, folic acid analogues, purine analogues, pyrimidine analogues, urea derivatives) and drugs for targeted therapy *(e.g.* kinase inhibitors, and monoclonal antibodies).

In one embodiment, the composition is not used in combination with an antineoplastic drug. In one embodiment, the composition is not used in combination with an antineoplastic drug as described in WO2012/059572, such as alkylating agents *(e.g.* nitrogen mustard analogues, nitrosoureas, alkyl sulfonates, platinum containing compounds, ethylemines, and imidazotetrazines), cytotoxic antibiotics *(e.g.* anthracyclines, actinomycins), plant alkaloids and other natural products *(e.g.* campthotecin derivatives, epipodophyllotoxins, taxanes, and vinca alkaloids), antimetabolites *(e.g.* cytidine analogues, folic acid analogues, purine analogues, pyrimidine analogues, urea derivatives) and drugs for targeted therapy *(e.g.* kinase inhibitors, and monoclonal antibodies). It will be understood that the term "combination" as used herein refers to the act of bringing together the composition (comprising AQCs) and the antineoplastic drug. Therefore, this term does not exclude the use of an antineoplastic drug at another time point during the course of cancer therapy, if said use is not with the aim of using the antineoplastic drug in combination with the claimed composition.

In one embodiment, the composition is for use as a monochemotherapy. References to "monochemotherapy" refer to the treatment of a cell proliferative disease, such as cancer, by the use of a single chemical drug. As discussed herein, the compositions of the invention have a chemotherapeutic effect of their own, without the need to be used in combination with other drugs, and therefore can be used as a monotherapy, in particular a monochemotherapy in the context of cancer treatment.

References to a "cell proliferative disorder" refer to a disorder resulting in the new, abnormal growth of cells or a growth of abnormal cells without physiological control. This can result in an unstructured mass, *i.e.* a tumour. In one embodiment, the cell proliferative disorder is a tumour and/or cancer. The compositions of the invention may be used to treat cell proliferative disorders including, but not limited to, primary tumours, metastases and precancerous conditions (pre-cancer stages).

Cancers may include, but are not limited to: spleen, colorectal and/or colon cancer, colon carcinomas, ovarian carcinomas, ovarian cancer, endometrial cancer, breast cancer, carcinomas of the uterus, lung cancer, stomach cancer, oesophageal cancer, liver cancer, carcinomas of the pancreas, kidney cancer, bladder cancer, prostate cancer, testicular cancer, bone cancer, thyroid cancer, skin cancer such as melanoma, sarcoma, Kaposi sarcomas, brain cancers such as glioma, medulloblastoma or neuroblastomas, blood cancers such as lymphomas and leukaemias, myosarcomas and head and neck carcinoma. In one embodiment, the cancer is selected from lung, breast, colon or brain cancer (in particular glioblastoma). In a further embodiment, the cancer is brain cancer, in particular brain cancer selected from glioma (such as glioblastoma multiforme, oligodendroglioma, ependymomas, brain stem glioma), craniopharyngioma, haemangioblastoma, malignant meningioma, pineal region tumours and vestibular schwannoma. In a yet further embodiment, the brain cancer is glioma, in particular glioblastoma.

The present invention has particular use in the treatment of cancers/tumours with a RAS mutation, such as a KRAS, NRAS or HRAS mutation, in particular KRAS mutations. Such mutations have been shown to cause oxidative stress in the tumour cells which results in high levels of ROS, for example see Shaw et al. (2011) PNAS 108(21): 8773-8778. As described herein, AQCs which consist of 5 atoms are potent in cells which comprise high levels of ROS. Therefore, in one embodiment, the cell proliferative disorder (e.g. cancer and/or tumour) comprises a RAS mutation. In a further embodiment, the RAS mutation is selected from a KRAS, NRAS or HRAS mutation, in particular a KRAS mutation. It will be understood that such cancers/tumours may also be referred to as a RAS mutant cancer, e.g. a KRAS, HRAS or NRAS mutant cancer or tumour. In a yet further embodiment, the RAS mutation is an activating mutation, i.e. the mutation causes increased or constitutive activity of a RAS protein. It is noted that in light of the mechanism of action of the AQCs of the invention, the composition may be used to treat RAS mutant cancer cells, regardless of the mutation. This is in contrast to current therapies which are specific to particular mutations of the RAS genes (in particular the KRAS gene).

The RAS family of proteins are GTPases which hydrolyse GTP to GDP allowing for activation of a number of downstream signalling pathways. For example, KRAS has been shown to be involved in the mitogen activated kinase pathway. Common mutations in KRAS reduce its intrinsic GTPase function, preventing hydrolysis of GTP to GDP, thus locking KRAS in its active state. This results in constitutive activation of downstream signalling pathways that can drive oncogenesis.

Many RAS mutations are known in the art and KRAS mutations are the most frequent oncogenic mutations in human cancer. A cancer comprises a RAS mutation if one or more of the cells in the cancer comprise(s) a RAS mutation. Subjects having RAS mutations may be identified by methods known in the art such as PCR, nucleic acid sequencing, allele-specific PCR methods, single-strand conformational polymorphism analysis, melt-curve analysis, probe hybridization, pyrosequencing (i.e. nucleotide extension sequencing), genotyping, and other sequencing methods (e.g. see Anderson (2011) Expert Rev Mol Diagn. 11(6): 635-642 and Ogino et al. (2005) J. Mol. Diagn. 7: 413-421). As shown herein, AQCs comprising 5 atoms had a toxic effect on the A549 cell line, which has been shown to comprise a KRAS mutation (such as KRAS G12S where the glycine residue at position 12 is mutated). Furthermore, cells comprising a HRAS mutation (HRasV12 where a mutation of the valine residue at position 12 was mutated) were more sensitive to the toxic effects of AQCs comprising 5 atoms compared to control cells.

It is estimated that 30% of all human cancers carry a RAS mutation. For example, 88% of pancreatic ductal adenocarcinomas, 52% of colorectal cancers, 43% of multiple myelomas, 32% of lung adenocarcinomas, 28% of melanomas, 25% of endometrial cancers, 13% of thyroid cancers, 12% of stomach cancers, 11% of acute myelogenous leukemias, 11% of bladder cancers, 6% of head and neck squamous cell carcinomas and 2% of breast cancers are believed to carry RAS mutations (data compiled from Cancer Cell Line Encyclopedia (CCLE); the International Cancer Genome Consortium (ICGC); and The Cancer Genome Atlas Data Portal (TCGA)). Therefore, in one embodiment, the cell proliferative disorder (in particular the cell proliferative disorder with a RAS mutation) is selected from pancreatic, colorectal, blood, lung, skin, endometrial, thyroid, stomach, bladder, head and neck or breast cancer. In a further embodiment, the cell proliferative disorder (in particular the cell proliferative disorder with a RAS mutation) is selected from pancreatic, colorectal, blood, lung, skin, endometrial, thyroid, stomach, bladder or head and neck cancer.

In one embodiment, the cell proliferative disorder is pancreatic cancer, e.g. pancreatic ductal adenocarcinoma, particularly a RAS mutant pancreatic cancer, e.g. a RAS mutant pancreatic ductal adenocarcinoma. In an alternative embodiment, the cell proliferative disorder is colorectal cancer, particularly a RAS mutant colorectal cancer. In an alternative embodiment, the cell proliferative disorder is blood cancer, e.g. multiple myeloma or acute myelogenous leukemia, particularly a RAS mutant blood cancer, e.g. a RAS mutant multiple myeloma or RAS mutant acute myelogenous leukemia. In an alternative embodiment, the cell proliferative disorder is lung cancer, e.g. non-small lung cell cancer such as lung adenocarcinoma, particularly a RAS mutant lung cancer, e.g. a RAS mutant non-small cell lung cancer, such as a RAS mutant lung adenocarcinoma. In an alternative embodiment, the cell proliferative disorder is skin cancer, e.g. melanoma, in particular a RAS mutant skin cancer, e.g. a RAS mutant melanoma. In an alternative embodiment, the cell proliferative disorder is endometrial cancer, in particular a RAS mutant endometrial cancer. In an alternative embodiment, the cell proliferative disorder is thyroid cancer, in particular a RAS mutant thyroid cancer. In an alternative embodiment, the cell proliferative disorder is stomach cancer, in particular a RAS mutant stomach cancer. In an alternative embodiment, the cell proliferative disorder is bladder cancer, in particular a RAS mutant bladder cancer. In an alternative embodiment, the cell proliferative disorder is head and neck cancer, e.g. head and neck squamous cell carcinoma, in particular a RAS mutant head and neck cancer, e.g. a RAS mutant head and neck squamous cell carcinoma.

The present invention has particular use in the treatment of cancers with low drug accessibility, such as large tumours with a low level of vascularity or brain tumours which are separated from the circulatory system by the blood-brain-barrier. This is due to the neutral charge and small size of the therapeutic AQCs which consist of just 5 atoms, allowing them to access areas in a tumour or cancer which are not easily accessible to traditional antineoplastic drugs.

Evidence is provided herein which shows the ability of AQCs consisting of 5 atoms to penetrate into the central hypoxic regions of multicellular tumour spheroids.

Preventing and treating metastasis of cancer is a key part of cancer treatment to prevent secondary cancers and relapse. It has been surprisingly found that the compositions of the invention have an additional beneficial effect of treating cancer metastases, as well as the primary tumour **(Figure 14).** Therefore, according to an aspect of the invention, there is provided the composition as described herein (in particular, a composition comprising atomic quantum clusters (AQCs) consisting of 5 zero-valent transition metal atoms), for use in the prevention and/or treatment of metastases, such as lymph node metastases, in particular to treat and/or prevent lung cancer metastasis. According to another aspect of the invention, there is provided the composition as described herein, for use in the prevention and/or treatment of lymph node metastasis of cancer.

In a further embodiment, the lymph node is a mediastinal node. Said mediastinal nodes are a group of lymph nodes located in the thoracic cavity of the body.

## Combination therapy

The compositions described herein may be used in combination with AQCs consisting of 3 zero-valent transition metal atoms. It has been found that the size of AQCs consisting of 3 zero-valent transition metal atoms enables them to intercalate into DNA and result in chromatin de-compaction. This can therefore be used to increase the susceptibility of treated cells to radiation and improve the effectiveness of radiation therapy.

In one embodiment, the composition (*i.e.* comprising AQCs consisting of 5 zero-valent transition metal atoms) and AQCs consisting of 3 zero-valent transition metal atoms are administered simultaneously. In this embodiment, the two agents are administered at the same time or at substantially the same time. They may also be administered by the same route and, optionally, in the same composition. Alternatively, they may be administered by different routes, *i.e.* separately, but at the same time or at substantially the same time.

In an alternative embodiment, the composition and the AQCs consisting of 3 zero-valent transition metal atoms are administered sequentially. In this embodiment, the two agents are administered at different times so that one of the agents is administered before the second agent. For example, the composition may be administered before or after the AQCs consisting of 3 zero-valent transition metal atoms. They may be administered by the same or different routes.

According to another aspect of the invention, there is provided a composition comprising AQCs consisting of 3 and 5 zero-valent transition metal atoms in combination with radiation therapy for use in the treatment of a cell proliferative disorder. In one embodiment, the composition consists of AQCs consisting of between 2 and 5 zero-valent transition metal atoms. In a further embodiment, the composition consists essentially of AQCs consisting of 3 and 5 zero-valent transition metal atoms.

The present inventors have surprisingly found that AQCs consisting of 5 atoms have a catalytic effect on thiol oxidation resulting in cell demise. Therefore, these AQCs may be used on their own as a cancer therapy and thus in one embodiment, the compositions described herein do not include additional antineoplastic drugs.

In one embodiment, the compositions of the present invention may include or be used in combination with additional therapeutic agents. Such agents may be active agents which are used in conjunction with cancer therapy, such as agents used as palliative treatments to ameliorate unwanted side effects. Therefore, in one embodiment, the additional therapeutic agent is an agent used as a palliative treatment. In a further embodiment, the palliative treatment is selected from the group consisting of: antiemetic agents, medication intended to alleviate pain such as opioids, medication used to decrease high blood uric acid levels such as allopurinol or rasburicase, anti-depressants, sedatives, anti-convulsant drugs, laxatives, anti-diarrhoeal drugs and/or antacids.

In one embodiment, the additional therapeutic agent is not an antineoplastic drug. In an alternative embodiment, the additional therapeutic agent is an antineoplastic agent. In one embodiment, the antineoplastic agent is selected from the group consisting of: alkylating agents (*e.g.* nitrogen mustard analogues, nitrosoureas, alkyl sulfonates, platinum containing compounds, ethylemines, and imidazotetrazines), cytotoxic antibiotics (*e.g.* anthracyclines, actinomycins), plant alkaloids and other natural products (*e.g.* campthotecin derivatives, epipodophyllotoxins, taxanes, and vinca alkaloids), antimetabolites (*e.g.* cytidine analogues, folic acid analogues, purine analogues, pyrimidine analogues, urea derivatives) and drugs for targeted therapy (*e.g.* kinase inhibitors, and monoclonal antibodies).

In one embodiment, the composition and the additional therapeutic agent are administered simultaneously. In this embodiment, the two agents are administered at the same time or at substantially the same time. They may also be administered by the same route and, optionally, in the same composition. Alternatively, they may be administered by different routes, *i.e.* separately, but at the same time or at substantially the same time.

In an alternative embodiment, the composition and additional therapeutic agent are administered sequentially. In this embodiment, the two agents are administered at different times so that one of the agents is administered before the second agent. They may be administered by the same or different routes.

In one embodiment, the composition is administered before the additional therapeutic agent. In an alternative embodiment, the composition is administered after the additional therapeutic agent.

**Radiation therapy**

Radiation therapy (also referred to as radiotherapy) uses high doses of radiation to damage cellular DNA and therefore kill cancer cells and shrink tumours. Such therapy may be in the form of an external beam or as internal radiation therapy. The choice of radiation therapy can depend on the type of cancer, size of the tumour, tumour location, as well as other factors, such as the age, general health and medical history of the patient and the other types of cancer treatment used.

Radiation therapy is administered to over 50% of all cancers, worldwide, and is of particular importance in developing and middle-income countries. However, effectiveness of radiation therapy is limited by various factors, including damage to healthy surrounding tissue, proximity of nearby organs and tumours developing radiation resistance. Therefore, there is a significant unmet need for agents to improve efficacy of radiation therapy.

Application of radiation therapy to cancer cells results in an increased production of ROS. As shown by the evidence provided herein, the effect of AQCs consisting of 5 atoms is potentiated in the presence of ROS. Therefore, the compositions of the present invention are particularly suited as therapeutic agents which enhance the effectiveness of radiation therapy.

According to an aspect of the invention, there is provided the composition as described herein, in combination with radiation therapy for use in the treatment of a cell proliferative disorder, such as cancer.

Radiation therapy (also referred to as radiotherapy) uses high doses of radiation to damage cellular DNA and therefore kill cancer cells and shrink tumours. Such therapy may be in the form of an external beam or as internal radiation therapy. The choice of radiation therapy can depend on the type of cancer, size of the tumour, tumour location and well as other factors, such as the age, general health and medical history of the patient and the other types of cancer treatment used.

According to an aspect of the invention, there is provided the use of a composition as described herein, as a radiotherapy sensitizing agent. According to another aspect of the invention, there is provided the use of the composition as described herein, as a radiation therapy sensitizing agent for proliferating cells. It will be understood that the term "radiotherapy sensitizing agent", also referred to as "radiosensitizers", refers to a drug which is used to enhance/increase the cytotoxic effect of radiation therapy. A cancer or tumour which is affected by radiation therapy is referred to as "radiosensitive".

According to another aspect, the invention provides a composition comprising atomic quantum clusters (AQCs) consisting of 5 zero-valent transition metal atoms for use as a radiation therapy desensitizing agent for non-proliferating cells.

Compositions comprising atomic quantum clusters (AQCs) consisting of 5 zero-valent transition metal atoms may be used to protect non-proliferating (such as non-dividing) cells from radiation therapy. It will be understood that the term "radiation therapy desensitizing agent", also referred to as "radiodesensitizers", refers to a drug which is used to reduce/decrease the cytotoxic effect of radiation therapy.

The compositions of the invention are therefore particularly advantageous when used in combination with radiation therapy because they have a dual effect of enhancing the effect of radiation therapy on proliferating cells *(i.e.* cancer cells) while also protecting non-proliferating cells *(i.e.* non-diseased cells) from harmful radiation.

References to "proliferation" will be understood by a person skilled in the art. As used herein "proliferating cells" refers to cells undergoing cell proliferation, *e.g.* cell growth and division. In particular, the invention is used to target cancer cells which have rapid, abnormal and/or uncontrolled cell proliferation. In one embodiment, the proliferating cells are cancer cells, precancer cells, or other abnormal, rapidly dividing cells in a subject. Also, as used herein, "non-proliferating cells" refers to cells which are not undergoing cell proliferation. These cells may also be described as "resting", "arrested", "quiescent", "non-dividing", "non-cycling" or "G0 cells". In one embodiment, the non-proliferating cells are non-cancerous cells.

Radiation therapy may be in the form of an external beam or as internal radiation therapy.

In one embodiment, the radiation therapy comprises external beam irradiation. External beam radiation therapy uses a radiation source that is external to the patient, typically either a radioisotope, such as Cobalt-60 (60Co), Cesium-137 (137Cs), or a high energy x-ray source, such as a linear accelerator machine (LINAC). The external source produces a collimated beam directed into the patient to the tumour site. The adverse effect of irradiating of healthy tissue can be reduced, while maintaining a given dose of radiation in the tumourous tissue, by projecting the external radiation beam into the patient at a variety of "gantry" angles with the beams converging on the tumour site.

Examples of external radiation therapy treatment, includes, but is not limited to, conformal radiotherapy, intensity modulated radiotherapy (IMRT), image guided radiotherapy (IGRT), 4-dimensional radiotherapy (4D-RT), stereotactic radiotherapy and radiosurgery, proton therapy, electron beam radiotherapy, and adaptive radiotherapy.

In an alternative embodiment, the radiation therapy comprises internal radiation therapy. In this embodiment, a radiopharmaceutical agent is administered to a patient and placed in the area to be treated. In one embodiment, the radiopharmaceutical agent comprises a radiation-emitting radioisotope. The radioisotopes are well known to a person skilled in the art and may comprise a metallic or non-metallic radioisotope.

Suitable metallic radioisotopes include, but are not limited to: Actinium-225, Antimony-124, Antimony-125, Arsenic-74, Barium-103, Barium-140, Beryllium-7, Bismuth-206, Bismuth-207, Bismuth212, Bismuth213, Cadmium-109, Cadmium-115m, Calcium-45, Cerium-139, Cerium-141, Cerium-144, Cesium-137, Chromium-51, Cobalt-55, Cobalt-56, Cobalt-57, Cobalt-58, Cobalt-60, Cobalt-64, Copper-60, Copper-62, Copper-64, Copper-67, Erbium-169, Europium-152, Gallium-64, Gallium-67, Gallium-68, Gadolinium153, Gadolinium-157 Gold-195, Gold-199, Hafnium-175, Hafnium-175-181, Holmium-166, Indium-110, Indium-111, Iridium-192, Iron 55, Iron-59, Krypton85, Lead-203, Lead-210, Lutetium-177, Manganese-54, Mercury-197, Mercury203, Molybdenum-99, Neodymium-147, Neptunium-237, Nickel-63, Niobium95, Osmium-185+191, Palladium-103, Palladium-109, Platinum-195m, Praseodymium-143, Promethium-147, Promethium-149, Protactinium-233, Radium-226, Rhenium-186, Rhenium-188, Rubidium-86, Ruthenium-97, Ruthenium-103, Ruthenium-105, Ruthenium-106, Samarium-153, Scandium-44, Scandium-46, Scandium-47, Selenium-75, Silver-10m, Silver-111, Sodium-22, Strontium-85, Strontium-89, Strontium-90, Sulfur-35, Tantalum-182, Technetium-99m, Tellurium-125, Tellurium-132, Thallium-204, Thorium-228, Thorium-232, Thallium-170, Tin-113, Tin-114, Tin-117m, Titanium-44, Tungsten-185, Vanadium-48, Vanadium-49, Ytterbium-169, Yttrium-86, Yttrium-88, Yttrium-90, Yttrium-91, Zinc-65, Zirconium-89, and Zirconium-95.

Suitable non-metallic radioisotopes include, but are not limited to: Iodine-131, Iodine-125, Iodine-123, Phosphorus-32, Astatine-211, Fluorine-18, Carbon-11, Oxygen-15, Bromine-76, and Nitrogen-13.

The type of radiation that is suitable for use in the present invention can vary. In one embodiment, the radiation therapy comprises electromagnetic radiation or particulate radiation. Electromagnetic radiation includes, but is not limited to, x-rays and gamma rays. Particulate radiation includes, but is not limited to, electron beams (beta particles), alpha particles, proton beams, neutron beams and negative pi mesons.

In one embodiment, the radiation therapy comprises brachytherapy. In brachytherapy, radiation sources are placed directly at the site of the cancer or tumour. This has the advantage that the irradiation only affects a very localized area thereby minimising exposure to radiation of healthy tissues. Furthermore, this allows the tumour to be treated with very high doses of localized radiation, whilst reducing the probability of unnecessary damage to surrounding healthy tissues.

In one embodiment, the brachytherapy comprises intracavitary treatment or interstitial treatment. Intracavitary treatment comprises placing containers that hold radiation sources into body cavities where the tumour is present or near to where the tumour is present. Interstitial treatment comprises placing containers that hold radioactive sources directly into a tumour or body tissue. These radioactive sources can stay in the patient permanently. Most often, the radioactive sources are removed from the patient after several days. Containers may comprise needles, seeds, wires, or catheters.

In one embodiment, the radiation therapy comprises systemic radioisotope therapy. In systemic radioisotope therapy, radiopharmaceutical agents comprising radioisotopes are delivered through infusion or ingestion. The administered radioisotopes may be targeted due to the chemical properties of the isotope, for example radioiodine which is preferentially absorbed by the thyroid gland. Targeting can also be achieved by conjugating the radioisotope to a targeting moiety, such as a molecule or antibody which binds to the target tissue. In one embodiment, the radiopharmaceutical agent comprises a radioactive conjugate. In a further embodiment, the radioactive conjugate is a radiolabelled antibody.

In one embodiment, the radiopharmaceutical agent is administered orally, parenterally, intraperitoneally, intravenously, intraarterially, transdermally, sublingually, intramuscularly, rectally, transbuccally, intranasally, via inhalation, vaginally, intra-occularly, locally, subcutaneously, intra-adiposally, intraarticularly or intrathecally. In one embodiment, the radiopharmaceutical agent is in a slow release dosage form.

The choice of radiation therapy can depend on the type of cancer, size of the tumour, tumour location and other factors, such as the age, general health and medical history of the patient and the other types of cancer treatment used.

In one embodiment, the composition and radiation therapy are applied simultaneously. In an alternative embodiment, the composition and radiation therapy are applied sequentially, preferably wherein the composition is applied prior to the radiation therapy. If the agents are administered separately, the radiation therapy may be administered while the composition is still effective, *i.e.* the composition and the radiation therapy are administered within a timeframe that will exert a synergistic or at least a combined effect upon administration to a patient. In one embodiment, the composition is administered not more than 6 hours prior to radiation therapy, such as between 1 and 6 hours prior to radiation therapy. In a further embodiment, the composition is administered about 6 hours, about 5 hours, about 4 hours, about 3 hours, about 2 hours or about 1 hour prior to radiation therapy.

In one embodiment, the therapeutic effect of the composition and the radiation therapy is synergistic. In one embodiment, the composition sensitizes cancer cells in the patient to radiation therapy.

In one embodiment, the compositions of the invention are able to improve the efficacy of the radiation therapy at least two-fold, such as three-fold, four-fold, five-fold or above, compared to the efficacy of the radiation therapy for the treatment of the disorder alone.

**Pharmaceutical compositions**

According to an aspect of the invention, there is provided a pharmaceutical composition comprising the compositions as described herein.

The compositions, and combinations where appropriate, may be formulated as a pharmaceutical composition, optionally comprising a pharmaceutically acceptable excipient, diluent or carrier. The carrier, diluent and/or excipient must be "acceptable" in the sense of being compatible with the other ingredients of the composition and not deleterious to the recipient thereof.

Examples of pharmaceutically acceptable carriers can include one or more of water, saline, phosphate buffered saline, dextrose, glycerol, ethanol and the like, as well as combinations thereof. Suitable pharmaceutical carriers, excipients or diluents are described in "Remington's Pharmaceutical Sciences" by E. W. Martin. Pharmaceutically acceptable carriers may further comprise minor amounts of auxiliary substances such as wetting or emulsifying agents, preservatives or buffers, which enhance the shelf life or effectiveness of the compositions of the invention. Pharmaceutical compositions may also include anti-adherents, binders, coatings, disintegrants, flavours, colours, lubricants, sorbents, preservatives, sweeteners, freeze dry excipients (including lyoprotectants) or compression aids.

Pharmaceutical compositions of the invention may be administered in a plurality of pharmaceutical forms of administrations, e.g. solid (such as tablets, pills, capsules, granules etc.) or liquid (such as solutions, suspensions, syrups, ointments, creams, gels or emulsions).

Pharmaceutical compositions of the invention can comprise a therapeutically effective amount. The therapeutically effective amount *(i.e.* the amount that produces an effect to help heal or cure the disorder to be treated) that may be administered to a subject will depend on multiple factors, such as the disease state, the age, sex, and weight of the individual, and the ability of the pharmaceutical composition to elicit a desired response in the individual. A therapeutically effective amount is also one in which any toxic or detrimental effects of the pharmaceutical composition of the invention, are outweighed by the therapeutically beneficial effects.

In one embodiment, the AQCs are present in an aqueous solution. In a further embodiment, the aqueous solution comprises dissolved oxygen, such as at least 2 times, or at least 3 times, the concentration of AQCs (in particular the concentration of AQCs comprising 5 zero-valent transition metal atoms) present in the mixture.

In one embodiment, the composition is administered (or is formulated for administration) by any suitable mode of delivery, such as intravenously, intraarterially, intracardially, intracutaneously, subcutaneously, transdermally, interperitoneally, intramuscularly, orally, lingually, sublingually, buccally, intrarectally or by enema.

The compositions of the invention may be administered directly to a target site *(i.e.* the site of the tumour) or systemically *(i.e.* into the circulatory system). Targeted administration has the advantage of focussing the therapeutic effect of the composition on the cancer or tumour to be treated. Such administration also minimises side-effects. However, the compositions of the invention are also suitable for systemic administration because the mode of action ensures that cellular apoptosis only occurs in cells with a high level of ROS. Levels of ROS are high in proliferating cells, *e.g.* cancerous cells. However, in normal, non-proliferating cells, levels of ROS are relatively low, therefore AQCs consisting of 5 atoms will have less of an effect on normal cells which helps to minimise adverse side effects.

In one embodiment, the composition is administered orally, intravenously or subcutaneously. In a further embodiment, the composition is administered orally. The advantage of the compositions of the present invention is that they may be depleted relatively quickly, therefore any side effects can be minimised because the AQCs do not persist in the body for an extended period.

A topical application is also possible *(e.g.* for the treatment of melanomas). A particular form of topical application consists of introducing the composition into a carrier system, in particular a drug delivery system, and implanting said carrier system into the cancerous tissues, wherein said carrier system then releases said composition specifically at the site of the cancerous tissue. In this way it is possible to avoid side effects, as may occur in the case of systemic administration, *i.e.* to reduce the overall strain on the body.

**Uses**

According to an aspect of the invention, there is provided the use of the composition as described herein for the treatment of a cell proliferative disorder.

According to an aspect of the invention, there is provided the use of the composition as described herein to treat and/or prevent metastasis of cancer. In one embodiment, the composition is used to treat and/or prevent lymph node metastasis of cancer. In a further embodiment, the composition is used to treat and/or prevent metastasis of lung cancer.

According to an aspect of the invention, there is provided the use of a composition comprising atomic quantum clusters (AQCs) consisting of 5 zero-valent transition metal atoms as a radiation therapy sensitizing agent for proliferating cells. Said agent may be used for the treatment of a cell proliferative disorder.

According to an aspect of the invention, there is provided the use of the composition as described herein, in combination with radiation therapy for the treatment of a cell proliferative disorder.

According to an aspect of the invention, there is provided the use of a composition as described herein, in the manufacture/preparation of a radiation therapy sensitizing agent for proliferating cells.

According to an aspect of the invention, there is provided the use of a composition comprising atomic quantum clusters (AQCs) consisting of 5 zero-valent transition metal atoms as a radiation therapy desensitizing agent for non-proliferating cells.

According to an aspect of the invention, there is provided the use of the composition as described herein, for the preparation of a pharmaceutical composition for the treatment of a cell proliferative disorder.

According to an aspect of the invention, there is provided the use of a composition as described herein, in the manufacture of a medicament for the treatment of a cell proliferative disorder.

**Atomic Quantum Clusters (AQCs)**

The AQCs described herein are stable, *i.e.* they conserve the number of atoms, and therefore their properties, overtime, so that they can be isolated and manipulated like any other chemical compound. The AQCs can be conserved for months, even years, without the need of an external stabilizer.

In one embodiment, the metal atoms are selected from silver (Ag), gold (Au), copper (Cu), platinum (Pt), iron (Fe), chromium (Cr), palladium (Pd), nickel (Ni), rhodium (Rh), lead (Pb), iridium (Ir), ruthenium (Ru), osmium (Os), cobalt (Co), titanium (Ti), vanadium (V) or any combination thereof. In a further embodiment, the metal atoms are selected from Ag, Au, Cu, Pt or any combination thereof. In a further embodiment, the metal atoms are selected from Ag, Cu or Pt. In a yet further embodiment, the metal atoms are Ag.

The mixture of AQCs may be synthesised by a variety of methods known in the art, for example those described in EP1914196, which is herein incorporated by reference.

The mixture may also be synthesised using the method described herein in Example 1. More specifically, there is provided a method of synthesising silver AQCs which comprises conducting the method in a three-electrode electrochemical cell comprising a hydrogen electrode as a reference electrode and two silver electrodes as the counter and working electrode, wherein the silver electrodes comprise a surface area which is greater than 5 cm$^2$, such as greater than 10 cm$^2$, for example about 17 cm$^2$. Clusters of 5 atoms may be obtained by applying a steadily increasing current for about 5 hours (300 minutes). From example, the increasing current can comprise: step (i) a current of about 200-300 μA (for example, about 250 μA), step (ii) a current of about 430-530 μA (for example, about 480 μA), step (iii) a current of about 800-1200 μA (for example, about 1000 μA/1 mA), step (iv) a current of about 2000-2400 μA (for example, about 2200 μA/2.2 mA) and/or step (v) a current of about 3800-4200 μA (for example, about 4000 μA/4 mA), or com-

binations of any of steps (i)-(v). In one embodiment, each step is conducted for at least 30 minutes, for example for about 1 hour. The silver electrodes may be polished prior and/or during synthesis, for example using sandpaper and/or alumina. The method may be conducted in purified, deaerated water, such as deaerated MilliQ water. Optionally, any excess Ag+ ions may be removed by the addition of NaCl and subsequent precipitation and filtration.

References to AQCs used herein, include those in the form of a hydrate, *i.e.* they have water molecules attached to the cluster via non-covalent bonding.

As described herein, the mechanism of increasing sulphur oxidation is unique to AQCs consisting of 5 metal atoms because the size of these clusters enables the interaction between the sulphur atom and ROS. Therefore, without being bound theory, it will be understood that compositions of the invention may not need to be completely free of AQCs consisting of other size clusters *(e.g.* clusters comprising less than and/or more than 5 metal atoms). In one embodiment, the composition comprises greater than about 50%, such as greater than about 55%, about 60%, about 65%, about 70%, about 75%, about 80%, about 85%, about 90%, about 95%, about 97%, about 99% of AQCs consisting of 5 zero-valent transition metal atoms. In particular, greater than about 95% of the AQCs present in the composition consist of 5 zero-valent transition metal atoms. In one embodiment, the composition consists essentially of AQCs consisting of 5 zero-valent transition metal atoms. In a further embodiment, the composition consists of AQCs consisting of 5 zero-valent transition metal atoms.

In one embodiment of the invention, the composition is substantially free of AQCs consisting of more than 5 zero-valent transition metal atoms, e.g. the composition may contain less than about 10 mol% (molar percentage based on the total AQC content of the composition), such as less than about 7 mol%, less than about 5 mol%, less than about 2 mol%, less than about 1 mol% or less than about 0.5 mol% of AQCs consisting of more than 5 zero-valent transition metal atoms.

In one embodiment of the invention, the composition is substantially free of AQCs consisting of less than 5 zero-valent transition metal atoms, e.g. the composition may contain less than about 10 mol% (molar percentage based on the total AQC content of the composition), such as less than about 7 mol%, less than about 5 mol%, less than about 2 mol%, less than about 1 mol% or less than about 0.5 mol% of AQCs consisting of less than 5 zero-valent transition metal atoms. AQCs consisting of less than 5 zero-valent transition metal atoms include AQCs consisting of 2, 3 or 4 zero-valent transition metal atoms.

In one embodiment, the composition is substantially free of metal ions. Metal ions are frequently a by-product during the synthesis of AQCs. These can be removed using, for example, NaCl. It will be understood that the reference to metal ions is with respect to ions of the transition metal contained in the AQCs.

In one embodiment, the composition contains less than about 20 mol%, such as less than about 15 mol%, 10 mol%, 5 mol%, 2 mol%, 1 mol% or 0.5 mol% of metal ions *(i.e.* free ions of the transition metal used to synthesise the AQCs).

According to a further aspect of the invention, there is provided a composition comprising atomic quantum clusters (AQCs) consisting of between 2 and 5 zero-valent transition metal atoms, which is substantially free (i.e. less than 20%, 15%, 10%, 5%, 2%, 1%) of AQCs consisting of more than 5 zero-valent transition metal atoms and/or metal ions. According to a further aspect of the invention, there is provided a composition comprising atomic quantum clusters (AQCs) consisting of 5 zero-valent transition metal atoms, which is substantially free *(i.e.* less than 20%, 15%, 10%, 5%, 2%, 1%) of AQCs consisting of more than 5 zero-valent transition metal atoms and/or less than 5 zero-valent transition metal atoms and/or metal ions.

Methods are known in the art for purifying compositions to remove AQCs consisting of more or less than 5 zero-valent transition metal atoms. For example, as described in Porto et al. (2018) Adv Mater. 30(33): e1801317. Such methods can include: (i) applying a solution comprising a mixture of AQCs to a separation medium, wherein said separation medium either binds or does not bind AQCs consisting of more than 5 zero-valent transition metal atoms; and (ii) isolating AQCs consisting of 5 zero-valent transition metal atoms.

In one embodiment, the separation medium is used in a chromatographic method. Chromatography is a method used to separate a mixture by passing a mobile phase comprising the mixture, through a stationary phase *(e.g.* comprising the separation medium described herein). The mixture is separated based on how the constituents of the mobile phase interact with the stationary phase. It will be understood that the fraction retained or discarded will depend on the content and whether the 5 zero-valent transition metal atoms are present. For example, if the separation medium retains AQCs consisting of more than 5 zero-valent transition metal atoms, then the eluate (which comprises AQCs consisting of 5 or fewer zero-valent transition metal atoms) is collected. Alternatively, if the separation medium retains AQCs consisting of 5 zero-valent transition metal atoms, then the eluate (which comprises AQCs consisting of more than and/or less than 5 zero-valent transition metal atoms) is discarded. In one embodiment, the separation medium is present in a chromatography column. Such chromatography columns are commercially available.

Separation mediums can comprise, for example, functional groups which bind to AQCs of particular sizes, such as a thiol group which binds to AQCs consisting of more than 3 zero-valent transition metal atoms. Alternatively, the functional group may comprise an aromatic group, such as a cyclic or polycyclic aromatic group. Separation mediums can also comprise, for example, deoxyribonucleic acid (DNA) which is substantially double stranded. It has been found that clusters of three metal atoms interact with DNA through intercalation which is strictly dependent upon the number of atoms in the cluster and independent of the type of base pairs (AT of GC) of the double helix. Therefore, DNA can be used to separate AQCs consisting of 3 zero-valent transition metal atoms.

In one embodiment, the separation medium is used in a dialysis method. Dialysis is a method of separating molecules based on their rates of diffusion through a semipermeable membrane. For example, the solution comprising a mixture of AQCs could be applied to a separation medium and then placed in a dialysis device *(e.g.* a dialysis cassette or dialysis tubing). Such dialysis cassettes, tubing or devices are commercially available. Dialysis membranes may be chosen with a molecular weight cut-off chosen according to the requirements of the separation *(e.g.* according to the molecular weight of the DNA used in the separation medium).

It will be understood that one or more of the purification methods disclosed herein may be conducted in combination and/or repeated one or more times. Conducting the purification method multiple times can increase the purification of the sample and allow the desired purification to be achieved.

**Methods of treatment**

According to an aspect of the invention, there is provided a method of preventing and/or treating a cell proliferative disorder comprising administering a therapeutically effective amount of a composition comprising atomic quantum clusters (AQCs) consisting of 5 zero-valent transition metal atoms, to a patient in need thereof. In one embodiment, said method does not comprise treating the patient with an additional antineoplastic drug.

According to an aspect of the invention, there is provided a method of preventing and/or treating a cell proliferative disorder comprising administering a therapeutically effective amount of the composition described herein, to a patient in need thereof.

According to an aspect of the invention, there is provided a method of treating a patient with a cell proliferative disorder comprising administering a composition as described herein. The embodiments described hereinbefore for the compositions may be applied to said methods of treatment *(e.g.* timing and mode of administration, formulation of composition, etc.).

According to an aspect of the invention, there is provided a method of preventing and/or treating metastasis of cancer comprising administering a composition as described herein. In one embodiment, the method prevents and/or treats lymph node metastasis of cancer. In a further embodiment, the method prevents and/or treats metastasis of lung cancer.

In one embodiment, the methods of treatment described herein additionally comprise treating the patient with radiation therapy, such as after administration of the composition. As described hereinbefore, the composition of the invention has particular use as a radiotherapy sensitizing agent.

In one embodiment, the composition is administered orally, intravenously or subcutaneously.

In one embodiment, the composition is administered simultaneously or prior to the radiation therapy.

In one embodiment, the method additionally comprises administering a therapeutically effective amount of a composition comprising AQCs consisting of 3 zero-valent transition metal atoms. In one embodiment, the composition comprising AQCs consisting of 3 zero-valent transition metal atoms is administered simultaneously or sequentially to the composition comprising AQCs consisting of 5 zero-valent transition metal atoms.

The patient may be any subject suffering from the disorder. In one embodiment, the patient is a mammal. In a further embodiment, the mammal is selected from a human or a mouse.

In one embodiment, the therapeutic effect of the composition and the radiation therapy is synergistic. In one embodiment, the composition sensitizes cancer cells in the patient to radiation therapy.

The method comprises administering a therapeutically effective amount of radiation. The amount of radiation used in radiation therapy is measured in Gray (Gy) units and varies depending on the type and stage of cancer being treated. Furthermore, the total dose of radiation may be divided into multiple, smaller doses known as "fractions" over a period of several days in order to minimise the negative side effects. A typical fractionation schedule for adults is 1.8 to 2 Gy per day, five days a week. A typical fractionation schedule for children is 1.5 to 1.8 Gy per day, five days a week.

In one embodiment, a total of at least about 10 Gy, such as 15 Gy, 20 Gy, 25 Gy, 30 Gy, 35 Gy, 40 Gy, 45 Gy, 50 Gy, 55 Gy, 60 Gy, 65 Gy, 70 Gy, 75 Gy, 80 Gy, 85 Gy, 90 Gy, 95 Gy or 100 Gy is administered to a patient in need thereof. The patient may receive radiation three, four or five times a week. An entire course of treatment may last from one to seven weeks depending on the type of cancer and the goal of treatment. In one embodiment, radiation therapy occurs over a period of at least 2, 3 or 4 weeks, such as 2-6 weeks, such as 2-4 weeks, or 5-8 weeks, in particular 5-7 weeks. For example, a patient can receive a dose of 2 Gy/day over about 30 days *(i.e.* 4-5 weeks).

In one embodiment, the radiation is administered at least once per day for five consecutive days per week. For example, the radiation is administered in at least about 2 Gy fractions at least once per day. In one embodiment, the radiation is administered every other day, three times per week. For example, radiation is administered in 10 Gy fractions every other day, three times per week.

In one embodiment, the radiation therapy is hypofractionated. Hypofractionation is a treatment regimen that delivers higher doses of radiation in fewer visits. In an alternative embodiment, the radiation therapy is hyperfractionated. Hyperfractionation is a treatment regimen that divides the total dose into more deliveries. It will be appreciated that many other factors are considered when selecting a dose, including whether the patient is receiving chemotherapy, patient comorbidities, whether radiation therapy is being administered before or after surgery, and the degree of success of surgery.

According to another aspect, the invention provides a method of preventing damage to non-proliferating cells in a patient undergoing radiation therapy, comprising administering a therapeutically effective amount of a composition comprising AQCs consisting of 5 zero-valent transition metal atoms to said patient prior to radiation therapy.

According to an aspect of the invention, there is provided a method of treating metastases, such as lymph node metastases, comprising administering a therapeutically effective amount of a composition comprising AQCs consisting of 5 zero-valent transition metal atoms, to a patient in need thereof, in combination with radiation therapy.

**Kits**

According to an aspect of the invention, there is provided a kit-of-parts comprising: the composition as described herein, optionally in admixture with a pharmaceutically acceptable adjuvant, diluent or carrier. The kit according to this aspect of the invention may be used in the treatment of a cell proliferative disorder.

In one embodiment, the kit may be used in combination with radiation therapy for the treatment of a cell proliferative disorder.

**Additional aspects**

According to an aspect of the invention, there is provided an apoptotic agent comprising AQCs consisting of 5 zero-valent transition metal atoms. The apoptotic agent may comprise the composition as described herein.

According to another aspect of the invention, there is provided a method of inducing thiol oxidation comprising administering the composition as described herein, optionally in combination with reactive oxygen species (ROS). As described herein, AQCs consisting of 5 atoms provide a catalytic bridge between ROS present in the cell and sulphur atoms in cysteine residues of proteins. Therefore, the composition of the invention can be used to enhance thiol oxidation. The addition of ROS would depend upon whether the target already contained ROS.

The present inventors have also provided evidence that AQCs consisting of 5 atoms have a strong bactericidal effect. Therefore, according to another aspect of the invention, there is provided a composition as described herein for use in the treatment of a disease caused by bacteria.

The invention will now be exemplified in the following, non-limiting examples.

**ABBREVIATIONS**

All units used herein are to be understood with their standard definition known in the art (unless specified otherwise).

| | |
|---|---|
| A549 | Human lung adenocarcinoma cell line |
| Ag | Silver |
| Ag5 | 5 Silver atoms |
| AQC | Atomic quantum cluster |
| ATCC | American Type Culture Collection |
| BBB | Blood-brain barrier |
| B-CLL | B-chronic lymphocytic leukaemia |
| CDDP | Cisplatin |
| Cul3 | Cullin 3 |
| Cys | Cysteine |
| DAPI | 4',6-Diamidino-2-phenylindole |
| DHE | Dihydroethidium |
| DMEM | Dulbecco's Modified Eagle Medium |
| DSMZ | Deutsche Sammlung von Mikroorganismen und Zellkulturen GmbH |
| DTT | Dithiothreitol |
| FBS | Fetal Bovine Serum |
| GBM | Glioblastoma multiforme |

GPx        Glutathione peroxidase
GRX1      Glutaredoxin 1
GSH       Glutathione
GSSG     Glutathione disulphide
$H_2O_2$     Hydrogen peroxide
HCT116    Human colorectal carcinoma cell line
HEK293    Human embryonic kidney 293 cell line
HMOX1    Heme oxygenase-1
Keap1     Kelch-like ECH-associated protein 1
Luc        North American Firefly Luciferase gene
MCF7     Human breast adenocarcinoma cell line
MCTS     Multicellular tumour spheroid
MEC-1     Human B-chronic lymphocytic leukaemia cell line
MM.1S    Human multiple myeloma cell line
MRE      Metal-responsive element
MT        Metallothioneins
MTT       3-(4,5-dimethylthiazol-2-yl)-2,5-diphenyltetrazolium bromide
MTF-1     Metal-responsive transcription factor 1 (MTF-1
NaCl      Sodium chloride
NAD(P)H  Nicotinamide adenine dinucleotide phosphate
Neh       Nrf2-ECH homology domain
NEM      N-ethylmaleimide
NQO-1    NAD(P)H quinone oxidoreductase
Nrf2      Nuclear factor (erythroid-derived 2)-like 2
$O_2$       Oxygen
PBS      Phosphate Buffered Saline
PFA      Paraformaldehyde
RL        Human Non-Hodgkin's lymphoma cell line
roGFP    Reduction-oxidation sensitive green fluorescent protein
ROS      Reactive oxygen species
TEM      Transmission electron microscopy
U87      Human glioblastoma multiforme cell line
XANES   X-ray absorption near edge structure

## MATERIALS AND METHODS

### Reagents and Materials

Unless otherwise specified, all reagents were purchased from Sigma Aldrich, Co., Spain. Silver sheets (99%) were purchased from Goodfellow Cambridge Ltd., Huntingdon, UK. Alumina nanoparticles (average size $\approx$ 50 nm) and cloth pads were purchased from Buehler, Düsseldorf, Germany.

Sandpaper (1,000 grit) was supplied by Wolfcraft España S.L, Madrid, Spain. All aqueous solutions were prepared with MilliQ-grade water using a Direct-Q8UV system from Millipore (Millipore Ibérica S.A., Madrid, Spain). Mica sheets (Grade V-1 Muscovite) were purchased from SPI Supplies, West Chester, PA, USA.

### X-ray absorption near edge structure (XANES)

S K-edge (2470 eV) XANES experiments were performed at de SXS beamline at the Laboratório Nacional de Luz Síncrotron (LNLS, Campinas, Brazil) which is equipped with an InSb (111) double crystal monochromator with slit aperture of 1 mm, to achieve a resolution of about 0.5 eV at the S K-edge. X-ray absorption spectra were recorded in fluorescence mode, collecting the emitted X-ray from the S K$\alpha$1,2 (at 2309.5 and 2308.4 eV, respectively) emission lines for each measured edge. Absorption experiments were performed either in a vacuum of 10-8 mbar at room temperature, and in a special liquid sample holder designed ad-hoc for the experiment with reactive oxygen species, at room temperature and atmospheric pressure. The photon energy was calibrated by assigning the value 2481.5 eV to the highest maximum of $Na_2S_2O_3$ (corresponding to the so-called inner sphere), in accordance with the criteria previously reported by Vairavamurthy (1998) Spectrochim. Acta. A. Mol. Biomol. Spectrosc. 54: 2009-2017. The final XANES spectra were obtained after background subtraction and normalization to the post edge intensity, following usual procedure described elsewhere. XANES quantification were performed with Athena software and subsequent analysis on Origin lab software. For glutathione characterization, a fraction of solution were deposited by drop casting on carbon disks (Ted Pella, Inc.) in order to have an Ag or S concentration in a detectable value. For thioredoxin samples, they were carefully mounted in a liquid sample holder, designed ad-hoc for this experiment. PBS solution was used as the solvent in all the reaction mixes with Thioredoxin, with the purpose to reproduce the same intracellular pH and ionic strength. The hydroxyl radical solutions were prepared by Fenton reaction, with $H_2O_2$ and $FeCl_2$.

Cell lines

Cell lines used in the development of this work included: A549 (human lung adenocarcinoma, DSMZ No: ACC 107), A549 Luc-C8 (Bioware®), MCF7 (human breast adenocarcinoma, DSMZ No: ACC 115), HCT116 (human colorectal carcinoma, ATCC No: CCL-247), HEK293 (Kidney, embryo from human), U87-luc (human glioblastoma multiforme, kindly provided by Joan Seoane), U251-Luc (human glioblastoma, kindly provided by Joan Seoane), MM.1S (human multiple myeloma, ATCC No: CRL-2974), RL (human non-Hodgkin's lymphoma, ATCC No: CRL-2261) and MEC-1 (human B-chronic lymphocytic leukemia, DSMZ, No: ACC 497). A549, A549-Luc, MCF7, U87-Luc, and HCT116 are derived from solid tumours and grow adherently as a monolayer while MM.1S, RL and MEC-1 are derived from hematological malignances and grow in suspension. A549, A549-Luc, U251-Luc and HEK293 cell lines were maintained in DMEM low glucose (D6046, Sigma); MCF7 and U87-Luc cell lines in DMEM high glucose (D5671, Sigma); HCT116, MM.1S and RL in Roswell Park Memorial Institute (RPMI) 1640 Medium (R-5886, Sigma) and MEC-1 cell line in DMEM/Nutrient Mixture F-12 Ham mixture 1:1. The medium was supplemented with 10% fetal calf serum and 1% (v/v) of L-glutamine, penicillin and streptomycin (Gibco). Modified cell lines (A549-Luc and U87-Luc) were supplemented with puromycin (1.3 μg/ml for A549-Luc and 5 μg/ml for U87-Luc) to select the stable transfected cells. All cell lines were cultured at 37°C in a humidified atmosphere in the presence of 5% $CO_2$ and 95% air.

Animals

Female Athymic nude mice weighing about 20-25 g and at the age of 8-12 weeks were used in the *in vivo* studies, which were supplied by Janvier Laboratories. The animals were acclimatized for at least 1 week before experimentation; they were housed in ventilated polypropylene cages at an average temperature of 22°C, with exposure to 12 hours of light and 12 hours of darkness each day. All mice received a standard laboratory diet of food and water ad libitum. The experiments were carried out according to the Rules of the Santiago de Compostela University Bioethics Committee and in compliance with the Principles of Laboratory Animal Care according to Spanish national law (RD 53/2013).

*In vitro* Cytotoxicity assay

Cytotoxicity was assessed by the MTT assay. Proliferating cells: A549 (4 x $10^3$ cells/well) and U251 (5 x $10^3$ cells/well) were seeded in 96-well plates. 24 hours later the medium was discarded and replaced with serum free media containing different concentrations of Ag5-AQCs (1.2-0.24 mg/L) for 1 hour and allowed to grow for 24 hours more in complete medium. B-CLL primary culture (4 x $10^4$ cells/well), was seeded in serum free medium and different concentrations of Ag5-AQCs (1.2-0.24 mg/L) were added immediately to the wells for 30 minutes. After that, complete medium was added and cells were allowed to grow for 24 hours. Non-proliferating cells (confluent or serum deprived): A549 cells (4 x $10^3$ cells/well) and U251 (5 x $10^3$ cells/well) were seeded in 96-well plates. Confluent cells were allowed to grow during 96 hours in medium-10% of FBS to reach the confluence. For serum deprived cells, 24 hours after seeding medium was replaced with medium-0.05% of FBS for 72 hours. In both conditions, non-proliferative state of cells was confirmed by flow cytometry. After, cells were treated with Ag5-AQCs (1.2-0.24 mg/L) for 1 hour in serum free medium and incubated in complete medium for 24 hours more.

Then, for all conditions tested, 10 μl of MTT solution (5mg/ml) was added to each well and incubated at 37°C protected from light. Four hours later, 100 μl of solubilization solution (SDS/0.1NHCl) were added and samples were incubated for 18 hours at 37°C. Absorbance was measured at 595 nm using a CLARIOstar® microplate reader. Similar protocols were followed using the MTT assay to measure cell viability with other cell types.

Radiometric measurement of GSH oxidation in living cells

PREMO Cellular Redox Sensor Grx-1-roGFP (Molecular Probes, P36242) is a genetically encoded sensor used to detect changes in the glutathione redox state in living cells. The sensor is based in the introduction of two cysteines into the β-barrel structure of the GFP protein. Under oxidizing conditions, the formation of a disulphide bonds alters the fluorescent properties of the biosensor resulting in a change in the emission intensity following excitation at two different wavelengths (400 and 488 nm). The ratio of emission intensities correlates with a change in the redox state of roGFP.

To analyze the changes in the oxidation state of GSH in the presence of Ag5-AQCs, 2.5x10⁴ A549 cells were seeded in 35 mm plate dishes (Mattek. P35GC-0-10-C) and transduced with the PREMO Cellular Redox Sensor Grx-1-roGFP just after the cells have been plated. Sensor volume was calculated following this equation:

$$\text{Sensor volume (ml)} = (\text{number of cells}) \times (\text{MOI}) / (1 \times 10^8).$$

Where "number of cells" is the number of cells seeded per dish; MOI is de number of viral particles per cell and $1 \times 10^8$ is the number of viral particles per ml of reagent.

According to the results obtained from the equation, 60 μl of PREMO Cellular Redox Sensor were added per millilitre of culture medium. Samples were incubated for 48 hours to obtain an optimal expression of the sensor and redox changes in living cells were motorized using the Leica TCS SP5 X confocal microscope. Ag5-AQCs (IC50) was added to the dish and images were taken every 10 seconds during 10 minutes. PREMO Cellular Redox Sensor was excited at 400 and 488 nm and emission was collected at 500-530 nm. The fluorescence intensity emitted by each cell was measured at both excitations and the ratio 400ex / 488ex was calculated. Images were processed using the ImageJ software.

Immunofluorescence

MTF-1: A549 (2.5 x 10⁴) cells were grown on glass coverslips in 24-well plates and treated with Ag5-AQCs (IC50) for 1 hour in serum free medium and then the medium was replaced with complete medium for 2 hours. HEK293 (8 x 104) cells were grown on glass coverslips in 24-well plates and treated with Ag5-AQCs (IC50), DTT (0.5 mM) or a combination of both, for 10 or 30 minutes in serum free medium and then the medium. After that cells were washed twice with PBS Ca2+/Mg2+ and fixed with Methanol/Acetone (dilution 1:1) for 10 minutes at -20°C. They were then blocked with PBS containing 10% FBS for 1 hour, washed twice with PBS and incubated with the primary antibody against MTF-1 (dilution 1:200) (sc-48775, Santa Cruz Biotechnology) overnight at 4°C. After that, cells were washed and incubated with the Alexa Fluor-594 goat anti-rabbit IgG secondary antibody (dilution 1:500) (A11037, Life Technologies) and Hoechst (dilution 1:1000) (Molecular Probes) at 0.25 μg/ml for 45 minutes. Coverslips with stained cells were mounted on glass slides with Fluoroshield Mounting Medium (F6182, Sigma). Images were obtained using the Leica TCS SP8 confocal microscope and analyzed using LasX software.

Nrf2: HEK293 cells (3 x 10⁴) were seeded on glass coverslips in 24-well plates overnight and treated with Ag5-AQCs (IC50), DTT (0.5 mM) or a combination of both, for 10 or 30 minutes in serum free medium. After treatment, cells were fixed in formalin solution (10%) for 30 minutes, washed twice with PBS Ca2+/Mg2+ and permeabilized with 0.5% Triton X-100 for 5-10 minutes. Then cells were washed again and blocked with PBS containing 1% BSA. Cells were subsequently incubated with the primary antibody against Nrf2 (dilution 1:100) (sc-722, Santa Cruz Biotechnology) for 2 hours at room temperature, washed twice with PBS and incubated with the Alexa Fluor-594 goat anti-rabbit IgG secondary antibody (dilution 1:250) (A11037, Life Technologies) and Hoechst (dilution 1:1000) (Molecular Probes) for 45 minutes. Negative controls without primary antibody were included in the analysis (data not shown). Coverslips with stained cells were mounted on glass slides with Fluoroshield Mounting Medium (F6182, Sigma). Images were obtained using the Leica TCS SP8 confocal microscope and analyzed using LasX software.

ROS measurement

Flow cytometry measurement was performed using the superoxide indicator dihydroethdium (DHE) (Molecular Probes, D11347). Cells were seeded in 12-well plate dishes and treated with Ag5-AQCs (IC50) in serum free medium. Cells were collected 30 minutes, 1, 2 and 3 hours after treatment, washed twice with cold PBS and incubated with DHE (3.17 mM) for 20 minutes at room temperature, protected from light. Stained cells were analyzed using the Guava EasyCyte flow cytometer with the InCyte software.

Multicellular tumour spheroids

A549 and U251 multicellular tumour spheroids (MCTSs) were generated by the hanging-drop method. 20 μl of a cell suspension containing 500 cells was dispensed in a 60-well mini tray (Nunc). After that, trays were inverted and incubated for 5 days under standard conditions. At day 5 trays were up-righted and spheroids were transferred to a 96-well plate coated with 50 μl of 1% agarose. Spheroids where then treated with Ag5-AQCs four times on alternate days and images of the spheroids were taken every day until the end of treatment using the Olympus IX51 microscope equipped with an Olympus DP72 camera and CellSens Imaging Software. Images were processed using ImageJ to measure spheroids area and differences in grey values as an indirect indicator of cellular density.

At the end of the experiment, spheroids were stained with Image-iT Green Hypoxia Reagent 5 $\mu$M (Molecular Probes, 114834) and Hoechst (1$\mu$g/$\mu$l) for 1 hour. Images of control and treated spheroids were taken on a Leica AOBS-SP5 confocal microscopy and analyzed using ImageJ software.

*In vivo* efficacy of Ag5-AQCs

A549luc orthotopic lung cancer model was developed following the protocol described by Borrajo et al. (2016) J. Control Release 238: 263-271. $1 \times 10^6$ A549Luc cells suspended in 50 $\mu$l of PBS were injected through the intercostal space into the left lung of athymic nude mice. Tumour evolution was followed by luciferin injection into the intraperitoneal cavity at a dose of 150mg/kg body weight approximately 5 minutes before imaging. Luciferase bioluminescence was imaged under vaporized isofluran anaesthesia using the IVIS LIVING IMAGE System (Caliper Life Sciences).

For Ag5-AQCs treatment, mice were divided into three groups (5 animals in each): the first group (control) underwent no treatment, the second group was treated with cisplatin (CDDP) (four single doses, 4 mg/kg) and the third group was treated with Ag5-AQCs (four single doses, 0.25 mg/kg). The drugs were applied intravenously through the tail vein on days 20, 22, 24 and 26 after the inoculation of the tumours. The mice were sacrificed the day 37. The lung and mediastinal lymph nodes were removed and luminescence was quantified per microgram of protein in body as described in Borrajo *et al.* (2016).

Histological analysis

Lungs were fixed in 10% neutral buffered formalin for 24 hours and embedded in paraffin. Sections 4 mm thick were mounted on FLEX IHC microscope slides (Dako-Agilent, Glostrup, Denmark) and heated at 60°C for 1 hour. The immunohistochemical technique was automatically performed using an AutostainerLink 48 (Dako-Agilent). After deparaffination and epitope retrieval in EnVision FLEX target retrieval solution (high pH) for 20 minutes at 97°C, the slides were allowed to cool in PT Link to 65°C and then in Dako wash buffer for 5 minutes at room temperature (RT). The immunostaining protocol included incubation at RT in: (1) EnVision FLEX peroxidase-blocking reagent (Dako-Agilent) for 5 minutes; (2) ready-to-use FLEX primary antibody (Dako-Agilent) anti-CK7 (clone OV-TL12/30), for 20 minutes; (3) EnVision FLEX/HRP (dextran polymer conjugated with horseradish peroxidase and affinity-isolated goat anti-mouse and anti-rabbit immunoglobulins) for 20 minutes; (4) substrate working solution (mix) (3,3'-diaminobenzidine tetrahydrochloride chromogen solution) (Dako-Agilent) for 10 minutes; and (5) EnVision FLEX haematoxylin (Dako-Agilent) for 9 minutes.

Sections were examined and photographed using an Olympus PROVIS AX70 microscope equipped with an Olympus DP70 camera.

Radiation treatment

A549 ($3 \times 10^4$ cells/well) and U251 ($3.5 \times 10^4$ cells/well) cells were seeded on a 24-well plate and incubated for 96 hours to reach confluence. Then, the medium was replaced with medium without FBS containing different dilutions of Ag5-AQCs (1:50, 1:75 and 1:100 for A549 and 1:150, 1:175 and 1:200 for U251). After pretreatment with Ag5-AQCs for 10 minutes, cells were irradiated with doses of 0-10 Gy using a Linear Accelerator from the Radiation Physics Laboratory at the Universidade de Santiago.

Statistical analysis

All statistical analyses were performed with GraphPad Prism Version 5.0 software (GraphPad Software, Inc., La Jolla, USA). The differences were considered significant for *$p < 0.05$, and very significant for *$p < 0.01$.

**EXAMPLE 1: Ag5-AQC synthesis method**

The synthesis of Ag5-AQC clusters was made at 25°C using a Biologic VMP3 potentiostat (Seyssinet-Oarsetm France). A Methrom thermally insulated three-electrode electrochemical cell was used with a hydrogen electrode as a reference and two Ag foils (17.5 $cm^2$ surface area) as counter and working electrodes. These electrodes faced each other and were separated at a distance of 3 cm. One first step of 1 hour at 250 $\mu$A, a second step of 1 hour of 480 $\mu$A, a third step of 1 hour of 1 mA, a four step of 1 hour of 2.2 mA, and two final steps of 30 minutes of 4 mA each at 25°C. Prior to the synthesis and also after 4 hours, and 4 and a half hours, both silver electrodes were polished with sandpaper followed by alumina (about 50 nm), washed thoroughly with MilliQ water and sonicated (2 steps of 5 minutes changing the water in each step). After sonication, and prior to the synthesis, an electrochemical cleaning was performed consisting in a step of 5 minutes at 250 mA in water.

Purification: The amount of unreacted ions in the solutions was estimated by Ag ion selective electrode (Hanna). An amount of NaCl 1.5 times the Ag ions concentration was used for the precipitation of Ag ions. The system was left during night at 25°C for complete precipitation.

Concentration: 16 syntheses were collected together (total 8 L), filtered through a membrane of 0.1 $\mu$m, and concentrated to a volume of 10 mL in a rotary evaporator (Heidolphlaborota 20) at 35°C (vacuum of $\approx$ 30 mbar). Finally, the solution was filtered through a membrane of 0.22microns and concentrated further in a vial to 2mL. The concentration of Ag5-AQCs at the end of the process of purification and concentration is around 30 mg/L, estimated by Flame Atomic Absorption Spectroscopy (performed with a Perkin-Elmer 3110 with a silver hollow cathode lamp Lumia from Perkin-Elmer (Madrid, Spain) (current 10 mA)). Mass spectra analysis shows mainly Ag5-AQCs species are present (approximately >50%).

Cluster samples were characterized by UV-Vis and fluorescence spectroscopy, AFM (atomic force microscopy), HRTEM (high resolution transmission electron microscopy), XANES and ESI-TOF (electrospray ionisation time-of-flight) mass spectrometry showing that the composition contains mainly clusters with N = 5 atoms (Ag5-AQCs).

## EXAMPLE 2: Model of interaction with Ag5

Theoretical models of the interaction of Ag5-AQCs with glutathione and thioredoxin show that the reaction is thermodynamically possible. Moreover, Ag5-AQCs interact selectively with a fundamental domain of thioredoxin, denominated "thioredoxin fold" and is found in both prokaryotic and eukaryotic proteins. Despite sequence variability in many regions of the fold, thioredoxin proteins share a common active site sequence with two reactive cysteine residues: Cys-X-Y-Cys, where X and Y are often but not necessarily hydrophobic amino acids. Without being bound by theory, Ag5-AQCs appear to interact with these two cysteine residues as shown in **Figure 1.**

## EXAMPLE 3: Ag5-AQCs promote sulphur oxidation

Ag5-AQCs promote sulphur oxidation in cysteine and glutathione as seen using X-ray absorption near edge structure (XANES) **(Figure 2)**. Moreover, this reaction is dose-dependent **(Figure 3)**. *E. Coli* thioredoxin is also shown to be oxidized in the presence of Ag5-AQCs **(Figure 4)**. As expected, pure thioredoxin molecule only show a peak at 2474.3 eV, consistently with S(-2) oxidation state, which correspond to their two cysteines groups. It is important to note that the reduced form of this molecule can be confirmed, before the catalytic treatment with Ag5-AQCs, because no signal of disulfide species with the characteristic splitting of about 1.5 eV in the range between 2473 and 2475 eV are observed. After treatment with AQCs, a strong peak associated to S+6 is clearly visible.

XANES analysis also shows the effect that different electron acceptors have on the Ag5-AQC mediated oxidation of thioredoxin **(Figure 5)**. From a biological point of view, it is of great importance to see that Ag5-AQCs potentiate the effect of oxygen, $H_2O_2$ and hydroxyl radicals (HO•) on sulphur oxidation, reaching oxidation states that are irreversible in biological systems. This links Ag5-AQC action to cell metabolism and tumour vascularisation **(Figure 6)**.

## EXAMPLE 4: Ag5-AQC have a bactericidal effect

Ag5-AQCs are bacteriostatic and bactericidal against *E. Coli*. The mechanism responsible is thought to be thiol oxidation. In fact, dithiothreitol (DTT), a thiol reducing agent, rescues *E. Coli* from Ag5-AQC action. The opposite is also true, *i.e.* Ag5-AQCs rescue *E. Coli* from DTT action as expected from to redox agents with opposed action. Clusters made from copper and platinum also have bactericidal activity.

In absence of DTT (0 mM), a low concentration (1.2 mg/L) of Ag5-AQCs kill the bacteria. When DTT is increased to 0.1 mM bacteria viability is partially restored. DTT at 10mM is toxic for bacteria, however Ag5-AQCs co-administration reverts the effect of DTT **(Figure 7)**.

## EXAMPLE 5: Effect of Ag5-AQCs on human cell lines

A panel of nine cell lines was used: A549 (human lung adenocarcinoma, DSMZ No.: ACC 107), A549 Luc-C8 (BIOWARE ®), MCF7 (human breast adenocarcinoma, DSMZ No.: ACC 115), HCT116 (human colorectal carcinoma, ATCC No.: CCL-247), HEK293: (Kidney, embryo from human), U87-luc (human glioblastoma multiforme, kindly provided by Joan Seoane), MM.1S (human multiple myeloma, ATCC No.: CRL-2974), RL (human Non-Hodgkin's lymphoma, ATCC No.: CRL-2261) and MEC-1 (human B-chronic lymphocytic leukaemia, DSMZ No.: ACC 497).

All cell lines were sensitive to Ag5-AQCs. In **Figure 8,** dose-response (0.24-1.2 mg/L) graphs are represented for various cell lines. Importantly, when DTT was co-administrated with Ag5-AQCs the toxic effect was reduced, indicating that Ag5-AQC effect is mediated by thiol oxidation.

Using A549 cell line, it was also found that clusters made of cooper show cytotoxic effects, see **Figure 9.**

The development of redox-sensitive GFP molecules allows the monitoring of redox status within live cells by fluorescence microscopy. The roGFP-Grx1 chimera is a genetically encoded sensor for measuring changes in thiol oxidation through two cysteines introduced into the β-barrel structure of the GFP protein. Disulphide formation between the cysteines leads to protonation of GFP and increases the 400 nm excitation spectra at the expense of the 488 nm excitation spectra. A549 cells were transduced with the sensor for 48 hours and changes in the fluorescence intensity were monitored by confocal fluorescence microscopy for 10 minutes after Ag5-AQC (IC50 - approximately 0.3 mg/L) treatment. While control cells do not modify their redox status over the time, the addition of Ag5-AQC to the sample led to a prompt oxidative response to the maximum signal after 6 minutes of treatment. A total of 34 randomly selected cells (at least 10 cells per experiment from a total of three experiments) were analysed, of which 20 cells showed a clear change in their redox state after exposure to Ag5-AQC. In addition, 13 of the remaining cells modified their oxidation state, although the effect is not as marked as in the 20 cells mentioned above. roGFP responds to levels of GSH/GSSG through electron exchange with glutaredoxin (GRX1), thus demonstrating Ag5-AQC effect on GSH **(Figure 10).**

## EXAMPLE 6: Ag5-AQCs act on critical thiols present in proteins

Metallothioneins (MTs) are a group of low molecular weight cysteine-rich intracellular metal binding proteins that play a critical role in protecting against oxidizing agents. MTs expression is under the control of the metal-responsive transcription factor 1 (MTF-1). Under normal conditions MTF-1 goes between the cytoplasm and the nucleus, but upon diverse stress it accumulates in the nucleus and binds to metal-responsive element (MRE) inducing the expression of MTs among other genes. Under physiological conditions, MTs bind zinc through the thiol group of its cysteine residues forming two zinc/thiolate clusters, but in conditions of oxidative stress zinc is released through the oxidation of the zinc/thiolate clusters leading to the formation of MT-disulphide. This MT-disulphide state can be reverted in a reduced environment, leading to the formation of MT-thiol which can associate with zinc ions to form MTs. This process constitutes the MT redox cycle, which plays a crucial role in the biological function of MTs.

It was reasoned that Ag5-AQCs could catalyse the conversion of MT-thiol to MT-disulphide with the consequent release of zinc, MTF-1 activation and translocation into the nucleus. To verify this, the location of MTF-1 after Ag5-AQC treatment was analysed. A549 cells were treated with Ag5-AQCs (IC50 - approximately 0.3 mg/L) and 2 hours later cells were fixed and stained with an antibody against MTF-1. Immunofluorescence images showed a clear nuclear accumulation of MTF-1 in treated cells with respect to control cells **(Figure 11a).** A total of 300 cells were counted for each condition of which 242 were positive for nuclear location of MTF-1 in Ag5-AQC-treated cells and 9 in controls. Moreover, microarray data obtained using the cell line MM.1S, after 4 hours of treatment with Ag5-AQCs showed that MTs genes were upregulated in response to Ag5-AQC treatment as expected from MTF-1 activation.

The Nrf2-Keap1 pathway is generally considered a major cellular defence pathway, which controls the expression of genes that have antioxidant functions within the cells. In basal conditions, Nrf2 is transcriptionally repressed by Keap1 in the cytoplasm which in turn facilitates the Cul3-mediated poly-ubiquitination of Nrf2 leading to its proteasomal degradation.

Keap1 contains 27 cysteines, some of which were reported to be the targets of electrophiles and oxidants that modify them facilitating the de-repression of Nrf2. Upon exposure to stresses, Keap1 is inactivated by direct modification of cysteine thiol residues, and subsequently Nrf2 is stabilized, avoiding proteasomal degradation and translocated into the nucleus to mediate the activation of a variety of genes implicated in the antioxidant response such as glutathione peroxidase (GPx), NAD(P)H quinone oxidoreductase (NQO-1), and heme oxygenase-1 (HMOX1). There are other mechanisms that regulate Nfr2 independently of Keap1, including the modification of cysteines in the Neh domain of Nrf2 which results in the nuclear accumulation of Nrf2. It was reasoned that Ag5-AQCs could be involved in the oxidation of the sulfhydryl groups in Keap1 or Nrf2 with the consequent release of Nrf2 and its translocation into the nucleus. Indirect immunofluorescence was used to assess the localization of Nrf2 protein in response to N-ethylmaleimide (NEM, positive control) and Ag5-AQCs. NEM is an alkene that is reactive toward thiols and is commonly used to modify cysteine residues in proteins and peptides. The A549 cell line presents mutations in the Keap1 gene resulting in an alteration of Keap1 activity which ceases to exert its repressive function on Nrf2 leading to a predominant localization of Nrf2 in the nucleus under normal conditions. Therefore, this cell line is not suitable for the study of Nrf2 cellular location. Instead, Human Embryonic Kidney 293 (HEK293) cells were exposed to NEM (100$\mu$M) and Ag5-AQC (IC50 - approximately 0.3 mg/L) for 30 minutes and then stained with specific antibodies against Keap1 and Nrf2. Ag5-AQC treatment caused an increase in Nrf2 protein staining (red staining) indicative of protein stabilization and nuclear accumulation (colocalization with blue, Hoechst staining) after 30 minutes compared to control cells, where Nrf2 is predominantly located in the cytoplasm **(Figure 11b).** As expected, NEM treatment increased the nuclear accumulation of Nrf2. It is clear than Ag5-AQCs and NEM share a similar pattern of staining (an increased expression of Nfr2 due to reduced degradation and increased nuclear localization), thus supporting Ag5-AQC action on thiols.

**EXAMPLE 7: Ag5-AQCs reduce multicellular-spheroids tumour growth**

Multicellular tumour spheroids (MCTSs) resemble many aspects of the pathophysiological conditions within human tumour tissue and are widely used for drug testing. MCTSs of A549 cells were therefore developed as an *ex vivo* tumour model to assess Ag5-AQC activity. The physiological state of cells in MCTS depends on its size; single MCTS of about 400-500 $\mu$m in diameter after 4-day incubation is frequently selected for drug testing. Therefore, MCTSs were selected according to these criteria and treated with Ag5-AQC (2.4 mg/L) four times (days 0, 2, 4 and 6, considering the first day of treatment as 0). Images of control and treated MCTSs were taken every day, from the day 0 to the day 7. Images showed that Ag5-AQC treatment reduce MCTSs growth **(Figure 12a)** as estimated by measuring MCTSs area using ImageJ.

The reduction in MCTS size was evident after the first dose of treatment and was maintained over time, being significantly different from day 3 **(Figure 12b)**. In addition, is noteworthy the existence of a clear region in the central part of the MCTSs treated with Ag5-AQC, that seems to be the result of a lower cellularity **(Figure 12a,** arrows). As described previously, large MCTSs (*i.e.* sizes above 600 $\mu$m) are characterized by the existence of heterogeneous cell subpopulations, with actively proliferating cells on the periphery and quiescent, hypoxic and necrotic cells in the inner regions. The existence of these clear regions in the MCTSs after Ag5-AQC treatment are thought to be related with the ability of Ag5-AQC to penetrate into the MCTSs reaching these central hypoxic regions by virtue of their small size and neutral charge.

To validate this hypothesis we assessed the levels of hypoxia in the tumouroid by using a fluorescent probe. As shown in **Figure 12c,** the levels of hypoxia increase in the inner part of the 1,000-cell tumouroid. Interestingly, exposure of the tumouroid to increasing concentration of Ag5-AQCs caused a dose-dependent reduction in hypoxic cells.

**EXAMPLE 8: Ag5-AQC action is potentiated by $H_2O_2$**

The above Examples provide evidence of the importance of the interaction of Ag5-AQC with $O_2$, $H_2O_2$ and hydroxyl radicals. Evidence is herein provided that the same is also true in cell cultures. Ag5-AQC cytotoxic activity was shown to be affected when cell respiration, and thus ROS levels, was decreased thereby modifying cell metabolism. In fact, A549 cells and U251 cells with reduced respiration (after allowing them to reach confluence) were less sensitive to Ag5-AQC action **(Figures 13A & 13B)**. As expected, serum starved A549 cells were also less sensitive to Ag5-AQCs than proliferating cells **(Figure 13C)**. The sensitivity was restored if a low dose of $H_2O_2$ was co-administered with Ag5-AQCs **(Figure 13D)**.

**EXAMPLE 9: Ag5-AQC *in vivo* effects**

*In vivo* effect of Ag5-AQC was tested in U87luc orthotopic glioma cancer model and in A549luc orthotopic lung cancer model that metastasizes to the mediastinal lymph nodes.

High-grade malignant glioma, glioblastoma multiforme (GBM), is the most aggressive and lethal form of brain tumours with a survival rate less than 5% after 5 years. One of the main limiting factors in the treatment of GBMs is the delivery of therapeutic agents to the brain across the blood-brain barrier (BBB). This highly restrictive, physiologic barrier prevents 98% of small-molecule drugs and virtually 100% of large-molecule drugs from reaching the central nervous system from blood circulation. The small size of AQCs coupled to their neutral charge at physiological pH favours, at least theoretically, its diffusivity in biological tissues. It was considered if these properties could allow Ag5-AQC to freely diffuse across the BBB and reach the tumour. To this aim, an U87luc orthotopic glioma model was developed to test the potential ability of Ag5-AQC to cross the BBB and reduce tumours.

After orthotopical implantation of U87luc cells, Ag5-AQC (0.5 mg/kg) were administered intravenously four times. The tumour growth was followed by measuring the bioluminescence of tumour cells in the brain during 14 days using the IVIS® Spectrum System. The results showed that, in the case of control animals tumour size increases exponentially throughout the experiment, while in animals treated with Ag5-AQC the growth of the tumour was reduced **(Figure 14a)**. Thus, these results show that Ag5-AQC are able to cross the BBB, reach the tumour and reduce its size.

Another limiting factor in cancer treatment is the occurrence of metastasis. Metastatic disease is largely incurable due to its systemic nature and the resistance of disseminated tumour cells to existing therapeutic agents. This explains why above 90% of mortality from cancer is attributable to metastases, but not the primary tumours from which these malignant lesions arise. The ability of Ag5-AQC to reduce or eliminate both primary tumour and metastases was evaluated using a previously described A549luc orthotopic lung cancer model that metastasizes to the mediastinal lymph nodes (Porto et al. (2018) Adv. Mater. e1801317). The A549 cell line is known to be a KRAS mutant cancer cell line. In this model the occurrence of metastasis to lymph nodes is well established and could be detected 13 days after the injection of tumour cells. Therefore, the evaluation of the Ag5-AQC effect started at day 20 after the implantation of A549luc cells, when lymph node metastases were already evident. Three groups were established: control untreated mice, mice treated with CDDP (4 mg/kg) as a positive control, and mice treated with Ag5-AQC (0.25 mg/kg). Treatment was administered intravenously four times (days 20, 22, 24 and 26) and the evolution of the tumour was monitored *in vivo* by measuring the bioluminescence of tumour cells in the lung during 37 days using the IVIS® Spectrum System. The results showed a significant reduction in tumour size in both CDDP and Ag5-AQC-treated mice compared to control animals, in which the tumour grew exponentially throughout the experiment **(Figure 14b)**. At day 37, animals were sacrificed because control mice showed evident signs of morbidity and the luciferase activity was quantified in order to measure the load of cancer cells. Ag5-AQC and CDDP treated-mice exhibited a significant reduction in luciferase activity in the primary tumour and in the mediastinal lymph nodes compared to control mice **(Figure 14c)**. The luciferase activity was then compared in mice treated with Ag5-AQC and in those treated with CDDP. In Ag5-AQC-treated mice the signal was clearly lower than in the others, both in the primary tumour and in the mediastinal lymph nodes **(Figure 14b,c)**. Immunohistochemical staining of lung sections with a monoclonal antibody against human cytokeratin, that specifically stain tumour cells, also corroborated these results **(Figure 14d)**. Moreover, Ag5-AQC treatment did not affect body weight of the animals, ruling out a severe toxicity of the compound **(Figure 14e)**.

Together, these results showed the ability of Ag5-AQC to reach both, primary tumour and metastases, and to significantly decrease the size without causing additional toxic effects. Therefore, Ag5-AQC offer a new approach that may improve the treatment of human tumours due to their capacity to cross the BBB and to reach and reduce metastases.

**EXAMPLE 10: Ag5-AQC effect on primary cultures from human tumours**

Ag5-AQC sensitivity was evaluated *ex vivo* in cells derived from B-chronic lymphocytic leukaemia (B-CLL) patients obtained from routine bone marrow cultures after the diagnosis was established. An assessment of the cytotoxic effect and ultrastructural morphological changes associated with Ag5-AQC treatment was performed in cells derived from 3 patients. B-CLL cells were cultured with different doses of Ag5-AQCs and cytotoxicity was assessed by MTT assay. In agreement with previous results obtained from established cell lines, a concentration-dependent reduction in cell viability was observed after 24 hours **(Figure 15a)**. Since Ag5-AQC in cancer cell lines increased $O_2^{\bullet-}$, we quantified changes in the levels of $O_2^{\bullet-}$ in B-CLL cells exposed to Ag5-AQC measuring DHE positive cells by flow cytometry. A significant increase, more than 2 folds, in DHE positive cells was observed 4 hours after treatment **(Figure 15b)**. Moreover, TEM images showed evident apoptotic morphological changes and the presence of disrupted mitochondria after Ag5-AQC treatment **(Figure 15c)**. Therefore, these results confirm the results obtained with cell lines and spheroids, discussed in the Examples above.

**EXAMPLE 11: Ag5-AQC effect on multiple myeloma tumour model**

The effect of Ag5-AQCs was tested in a multiple myeloma xenograft MM.1S mouse tumour model. Mice (n=4 for each treatment group) were treated with saline solution (control), 0.125 mg/kg Ag5-AQCs or 0.25 mg/kg Bortezomib (a proteasome inhibitor approved in US and Europe for treating multiple myeloma). Tumour volume was monitored over several days and the results are shown in **Figure 16.** The results show that Ag5-AQC treatment had equivalent efficacy to Bortezomib, even without optimized dosing.

**EXAMPLE 12: Ag5-AQCs causes cell death preferentially in Ras-transformed cells**

It has been widely reported that activated oncogenes cause an accumulation in reactive oxygen species (Irani et al. (1997) Science). It was reasoned that transformed cells could be preferentially killed by treatment with Ag5-AQCs. To test this hypothesis, a doxycycline-inducible activated allele of H-Ras (RasV12) was introduced into non-transformed immortalized mouse fibroblasts (W3T3) and the effects of Ag5-AQCs upon oncogene induction was analysed. As shown in **Figure 17B,** Ag5-AQCs caused a decrease in survival in W3T3 in a dose-response manner. Interestingly, cells expressing RasV12 were more sensitive to the toxic effects of Ag5-AQCs, with a significant decrease in viability compared to non-induced cells (control) in all tested doses of AQCs.

This selective killing was reversed by DTT treatment suggesting that Ras-induced ROS accumulation was responsible for the differential effect. This preferential effect of Ag5-AQCs on oncogene-transformed cells provide evidence for the use of Ag5-AQCs as antineoplastic agents especially in RAS mutant cancers.

**EXAMPLE 13: Effect of AQCs in combination with radiation therapy**

The effect of Ag5-AQC treatment on cells treated with radiation was tested. A549 cells and U251 cells were treated with different dilutions of Ag5-AQCs and immediately irradiated with different doses of radiation (0-10 Gy).

Cell survival was measured using a clonogenic assay as described in Franken et al. (2006) Nat. Protocol. 1(5): 2315-2319. Briefly, after irradiation, cells were trypsinized and counted using the TC20 automated cell counter (Biorad).

100 cells from each sample were seeded in a 6-well plate in triplicate and incubated at 37°C in a humidified atmosphere for 1 week to allow the formation of macroscopic colonies. Then, cells were fixed and stained with crystal violet. Colonies with more than 50 cells were counted. Surviving fraction (SF) was calculated after correction for plating efficiency of control cells. Results are shown in **Figures 18A** and **19A.** The results show that administration of Ag5-AQCs increases the cell killing effect of radiotherapy.

DNA damage was also measured in irradiated cells. After irradiation, cells were trypsinized fixed with PFA (0.04%) and stained with the anti-pH2AX antibody (Millipore, Product No: 16-202A) as described in Muslimovic et al. (2008) Nat. Protocol. 3: 1187-1193. Phosphorylated histone H2AX (pH2AX) expression is a marker of DNA damage (Sharma et al. in DNA Repair Protocols, (Ed: L. Bjergbæk), Springer Science, New York, 2012, Ch. 40). Stained cells were analyzed on the Guava EasyCyte flow cytometer using the InCyte program (Millipore). Results are shown in **Figures 18B** and **19B.**

The effect of AQCs consisting of 5 zero-valent transition metal atoms may be further tested in two different models to determine the effect with radiation therapy. A) *Ex-vivo* studies, using multicellular tumour spheroids (MCTSs): MCTSs of U251-luc cells may be obtained by the hanging drop method. B) *In vivo* model: an orthotopic glioblastoma cancer model may be developed by the injection of U251-luc cells (a human glioblastoma cell line which carries the luciferase gene to allow the *in vivo* imaging of the growing tumours) into the brain of athymic mice.

**CONCLUSION**

Described herein is the effect of Ag5-AQCs in the oxidation of proteins with a high cysteine content and accessible thiol groups such as metallothioneins or glutharedoxin. It was proposed that due to their small size, Ag5-AQCs should exhibit an excellent penetration into tumour tissue. Experiments in MTCSs showed how Ag5-AQCs penetrate to the inner regions killing the hypoxic cells in these areas. Orthotopic lung cancer model that metastasizes to the mediastinal lymph nodes reveal the ability of Ag5-AQCs to reach tumours *in vivo.* A reduction in tumour sizes was observed both, lung primary tumour and mediastinal lymph nodes metastases. These results highlight the ability of Ag5-AQCs to reach and reduce metastases constituting an innovative tool to solve two of the major problems in cancer treatment.

All patents and patent applications referred to herein are incorporated by reference in their entirety. Furthermore, all embodiments described herein may be applied to all aspects of the invention.

**Claims**

1. A composition comprising atomic quantum clusters (AQCs) consisting of 5 zero-valent transition metal atoms for use in the treatment of a cell proliferative disorder.

2. The composition for use according to claim 1, in combination with radiation therapy.

3. The composition for use according to claim 2, for simultaneous or sequential administration to the radiation therapy.

4. The composition for use according to claim 2 or claim 3, wherein the radiation therapy is external beam radiation therapy.

5. The composition for use according to any one of claims 1 to 4, wherein said composition is not used in combination with an antineoplastic drug.

6. The composition for use according to any one of claims 1 to 5, wherein said AQCs are the sole active ingredient of the composition.

7. The composition for use according to any one of claims 1 to 6, for use as a monochemotherapy.

8. The composition for use according to any one of claims 1 to 7, wherein the metal atoms are selected from Ag, Au, Cu, Pt, Fe, Cr, Pd, Ni, Rh, Pb, Ir, Ru, Os, Co, Ti, V or any combination thereof.

9. The composition for use according to claim 8, wherein the metal atoms are selected from Ag, Au, Cu, Pt or any combination thereof.

10. The composition for use according to claim 8 or claim 9, wherein the metal atoms are Ag.

11. The composition for use according to any one of claims 1 to 10, wherein the cell proliferative disorder is a tumour and/or cancer.

12. The composition for use according to claim 11, wherein the cancer is selected from brain, lung, breast or colon cancer.

13. The composition for use according to claim 12, wherein the cancer is selected from brain cancer.

14. The composition for use according to any one of claims 1 to 13, wherein the cell proliferative disorder comprises a RAS mutation.

15. The composition for use according to claim 14, wherein the RAS mutation is a KRAS mutation.

16. The composition for use according to any one of claims 1 to 15, wherein the composition comprises a pharmaceutically acceptable excipient, diluent or carrier.

17. The composition for use according to any one of claims 1 to 16, in combination with AQCs consisting of 3 zero-valent transition metal atoms.

18. The composition for use according to any one of claims 1 to 17, which is: (i) substantially free of AQCs consisting of more than 5 zero-valent transition metal atoms, (ii) substantially free of AQCs consisting of less than 5 zero-valent transition metal atoms, and/or (iii) substantially free of metal ions.

19. The composition for use according to claim 18, wherein greater than about 95% of the AQCs present in the composition consist of 5 zero-valent transition metal atoms.

20. The composition according to any one of claims 1 to 19, for use in the prevention and/or treatment of metastasis of cancer.

21. The composition according to claim 20, for use in the prevention and/or treatment of lymph node metastasis of cancer.

22. The composition according to claim 20 or claim 21, for use in the prevention and/or treatment of metastasis of lung cancer.

23. The composition for use according to any one of claims 1 to 22, which is administered orally, intravenously or subcutaneously.

24. Use of the composition according to any one of claims 1 to 23, for the preparation of a pharmaceutical composition for the treatment of a cell proliferative disorder.

25. Use of a composition comprising atomic quantum clusters (AQCs) consisting of 5 zero-valent transition metal atoms as a radiation therapy sensitizing agent for proliferating cells.

26. A method of preventing or treating a cell proliferative disorder comprising administering a therapeutically effective amount of the composition according to any one of claims 1 to 23, to a patient in need thereof.

27. A method of preventing or treating a cell proliferative disorder comprising administering a therapeutically effective amount of a composition comprising atomic quantum clusters (AQCs) consisting of 5 zero-valent transition metal atoms, to a patient in need thereof, wherein said method does not comprise treating the patient with an additional antineoplastic drug.

28. A method of preventing or treating a cell proliferative disorder comprising administering a therapeutically effective amount of a composition comprising atomic quantum clusters (AQCs) consisting of 5 zero-valent transition metal atoms, to a patient in need thereof, in combination with radiation therapy.

29. The method according to claim 28, wherein the composition is administered simultaneously or prior to the radiation therapy.

30. The method according to claim 28 or claim 29, which comprises administering a therapeutically effective amount of a composition comprising AQCs consisting of 3 zero-valent transition metal atoms.

31. The method according to claim 30, wherein the composition comprising AQCs consisting of 3 zero-valent transition metal atoms is administered simultaneously or sequentially to the composition comprising AQCs consisting of 5 zero-valent transition metal atoms.

32. The method according to any one of claims 26 to 31, wherein the composition is administered orally, intravenously or subcutaneously.

-167 kJ/mol

FIGURE 1

**FIGURE 2**

**FIGURE 3**

**FIGURE 4**

**FIGURE 5**

**FIGURE 6**

**FIGURE 7**

**FIGURE 8**

FIGURE 8 (contd.)

FIGURE 9

**FIGURE 10**

a

FIGURE 11

b

|  | Control | NEM | Ag5-AQCs |
|---|---|---|---|
| Hoechst | | | |
| Nrf2 | | | |
| Keap1 | | | |
| Merge | | | |

FIGURE 11 (contd.)

FIGURE 12

C

FIGURE 12 (contd.)

A)

B)

FIGURE 13

C)

D)

FIGURE 13 (contd.)

**FIGURE 14**

FIGURE 14 (contd.)

e

FIGURE 14 (contd.)

FIGURE 15

**FIGURE 15 (contd.)**

FIGURE 15 (contd.)

**FIGURE 16**

**FIGURE 17**

**FIGURE 18**

A)

B)

FIGURE 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012059572 A

- EP 1914196 A

### Non-patent literature cited in the description

- **SHAW et al.** *PNAS,* 2011, vol. 108 (21), 8773-8778
- **ANDERSON.** *Expert Rev Mol Diagn.,* 2011, vol. 11 (6), 635-642
- **OGINO et al.** *J. Mol. Diagn.,* 2005, vol. 7, 413-421
- **PORTO et al.** *Adv Mater.,* 2018, vol. 30 (33), e1801317
- **VAIRAVAMURTHY.** *Spectrochim. Acta. A. Mol. Biomol. Spectrosc.,* 1998, vol. 54, 2009-2017

- **BORRAJO et al.** *J. Control Release,* 2016, vol. 238, 263-271
- **PORTO et al.** *Adv. Mater.,* 2018, e1801317
- **IRANI et al.** *Science,* 1997
- **FRANKEN et al.** *Nat. Protocol.,* 2006, vol. 1 (5), 2315-2319
- **MUSLIMOVIC et al.** *Nat. Protocol.,* 2008, vol. 3, 1187-1193
- **SHARMA et al.** DNA Repair Protocols. Springer Science, 2012